# EUROPEAN PATENT SPECIFICATION

(11) **EP 3 733 177 B1**
(45) Date of publication and mention of the grant of the patent: **06.07.2022**
(21) Application number: 18893697.5
(22) Date of filing: 26.12.2018
(51) Int. Cl.: A61K 31/4418, A61P 27/02, A61P 27/04, A61P 43/00

(54) **COMPOSITION FOR PROTECTING CORNEA**
ZUSAMMENSETZUNG FÜR HORNHAUTSCHUTZ
COMPOSITION POUR LA PROTECTION DE LA CORNÉE

(30) Priority: 27.12.2017 JP 2017251839
(43) Date of publication of application: 04.11.2020
(73) Proprietor: Kyoto University, Kyoto-shi, Kyoto 606-8501 (JP); Kyoto Prefectural Public University Corporation, Kyoto-shi, Kyoto 602-8566 (JP); The Doshisha, Kyoto 602-8580 (JP)
(72) Inventor: IKEDA, Hanako, Kyoto-shi, Kyoto 606-8501 (JP); KAKIZUKA, Akira, Kyoto-shi, Kyoto 606-8501 (JP); KINOSHITA, Shigeru, Kyoto-shi, Kyoto 602-8566 (JP); NAKAMURA, Takahiro, Kyoto-shi, Kyoto 602-8566 (JP); NAGATA, Maho, Kyoto-shi, Kyoto 602-8566 (JP); KOIZUMI, Noriko, Kyotanabe-shi, Kyoto 610-0394 (JP); OKUMURA, Naoki, Kyotanabe-shi, Kyoto 610-0394 (JP)
(74) Representative: Hoffmann Eitle
(86) International application number: PCT/JP2018/047749
(87) International publication number: WO 2019/131720

(56) References cited:
- EP-A1- 2 623 494
- EP-A1- 2 959 903
- WO-A1-2012/014994
- WO-A1-2012/043891
- WO-A1-2017/222042
- HANAKO OHASHI IKEDA ET AL: "Novel VCP modulators mitigate major pathologies of rd10, a mouse model of retinitis pigmentosa", SCIENTIFIC REPORTS, vol. 4, no. 1, 6 August 2014 (2014-08-06), pages 1-9, XP055624208, DOI: 10.1038/srep05970
- HASEGAWA TOMOKO ET AL: "Neuoroprotective efficacies by KUS121, a VCP modulator, on animal models of retinal degeneration", SCIENTIFIC REPORTS, vol. 6, 9 August 2016 (2016-08-09), XP002803967, ISSN: 2045-2322
- STASTNA,M. et al.: "Analysis of protein composition of rabbit aqueous humor following two different cataract surgery incision procedures using 2- DE and LC-MS/MS", Proteome Science, vol. 9, no. 8, 2011, pages 1-15, XP021088624,
- Nagata Maho et al.: "O05: Corneal tissue protective effect of VCP ATPase inhibitor", Japan Cornea Conference 2018 (42nd Japan Cornea Society / 34th Annual Meeting of Keratoplasty Society of Japan); February 15 17, 2018, vol. 42, February 2018 (2018-02), page 46, XP009521884,

## Description

### TECHNICAL FIELD

The disclosure relates to a composition for protecting a cornea.

### BACKGROUND

A cornea is one of the tissues constituting an eye. Cornea endothelial cells located at the innermost layer of the cornea work as a pump and are essential for maintaining the corneal transparency. The corneal endothelium has no proliferative capacity and is not regenerated once damaged. When the corneal endothelium is damaged, endothelial cells surrounding the damaged part expand to cover it. However, if the cells are too decreased, the corneal transparency cannot be maintained and the visual acuity is extremely deteriorated.

The corneal endothelium may be damaged by intraocular surgery such as cataract surgery or glaucoma surgery, rapid ocular hypertension, inflammation, trauma, and a disease such as Fuchs endothelial corneal dystrophy. If the damage progresses, it is necessary to compensate for the decreased corneal endothelial cells by corneal transplantation (e.g., penetrating keratoplasty or Descemet stripping endothelial keratoplasty). No effective therapy is available for regenerating the corneal endothelial cells or suppressing the decrease of the cells.

Corneal epithelial cells turn over every day by virtue of stem cells present at the corneal limbus (boundary between the cornea and the conjunctiva). However, once the stem cells are exhausted due to, for example, trauma or an inflammatory conjunctival disease, corneal epithelial cells are not sufficiently supplied and the corneal epithelial is defected. As a result, the corneal transparency cannot be maintained, the cornea epithelium turns to a part of the conjunctiva, and the visual acuity is impaired. Additionally, corneal epithelial disorders are often accompanied by strong ocular pain. Though an anti-inflammatory agent could be used for suppressing the decrease of the corneal epithelial cells and the corneal epithelial stem cells, the effect is only limited.

A cornea has to be protected in ocular surgery or corneal implantation. Some products for ocular irrigation in ocular surgery are currently available, which contain glutathione for protecting corneal endothelial cells and retinal cells. A corneal preservation solution such as Optisol-GS is generally used for preserving a cornea donated for corneal transplantation, but the preservation effect does not last longer than about two weeks.

Accordingly, protection of corneas as well as treatment or prevention of corneal diseases is required in various situations.

Certain 4-amino-naphthalene-1-sulfonic acid derivatives having a VCP (valosin-containing protein) ATPase inhibitory activity (Patent Literature 1) are known to be effective in treatment or prevention of ocular diseases that affect retinal tissues and optic nerves in a posterior eye segment, such as glaucoma, retinitis pigmentosa, age-related macular degeneration, and ischemic ocular diseases (Patent Literatures 2-4, Non-Patent Literatures 1 and 2). However, any effect of the agents on a cornea has not been known.

### REFERENCES

### PATENT LITERATURE

[Patent Literature 1] WO2012/014994
[Patent Literature 2] WO2012/043891
[Patent Literature 3] WO2014/129495
[Patent Literature 4] WO2015/129809

### NON-PATENT LITERATURE

[Non-Patent Literature 1] Hanako Ikeda et al., Sci Rep 4, 5970, 2014
[Non-Patent Literature 2] Noriko Nakano et al., Heliyon 2, e00096, 2016

### SUMMARY

The present invention is defined in the appended claims.

An object of the disclosure is to provide a composition for protecting a cornea.

The inventors have found that VCP inhibitors protect corneal endothelial cells and corneal epithelial cells.

Accordingly, an aspect of the disclosure provides a composition for protecting a cornea comprising a compound of formula (I): 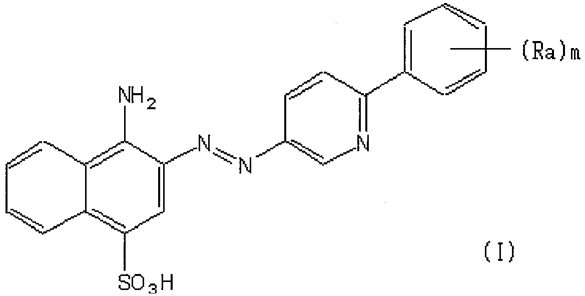 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, prodrug, pharmaceutically acceptable salt or solvate thereof.

Another aspect of the disclosure provides a pharmaceutical composition for treating or preventing a corneal disease comprising a compound of formula (I), or an ester, oxide, prodrug, pharmaceutically acceptable salt or solvate thereof.

Another aspect of the disclosure provides a composition for ocular irrigation comprising a compound of formula (I), or an ester, oxide, prodrug, pharmaceutically acceptable salt or solvate thereof.

Another aspect of the disclosure provides a composition for preserving a corneal graft comprising a compound of formula (I), or an ester, oxide, prodrug, pharmaceutically acceptable salt or solvate thereof.

According to the disclosure, a composition protecting a cornea is provided. Specifically, a pharmaceutical composition for treating or preventing a corneal disease is provided. Alternatively, a composition for ocular irrigation or preserving a corneal graft which has an improved corneal protection effect is provided.

### BRIEF DESCRIPTION OF DRAWINGS

Fig. 1 is the phase-contrast images of rabbit corneal endothelial cells cultured in the presence of antimycin or oligomycin, indicating that KUS121 protects the cells.
Fig. 2 shows the phase-contrast images of rabbit corneal endothelial cells cultured in the absence of glucose, indicating that KUS121 protects the cells.
Fig. 3 shows the measurements of the intracellular ATP concentrations in rabbit corneal endothelial cells cultured in the presence of antimycin or oligomycin or in the absence of glucose, indicating that the intracellular ATP concentration is kept from decreasing in the presence of KUS121.
Fig. 4 shows the phase-contrast images demonstrating cell morphology and apoptosis of immortalized human corneal endothelial cells cultured in the presence of thapsigargin, indicating that KUS121 suppresses the cell damage.
Fig. 5 shows the results of western blot analysis of cleaved caspase 3 and PARP in immortalized human corneal endothelial cells cultured in the presence of thapsigargin, indicating that apoptosis is suppressed in the presence of KUS121.
Fig. 6 shows the HE-stained images of tissue sections of rabbit corneas preserved in Optisol-GS for one to four weeks, indicating the morphological changes of the corneal tissues are suppressed in the presence of KUS121 or KUS187.
Fig. 7 shows the measurements of the central corneal thicknesses of rabbit corneas preserved in Optisol-GS for one to four weeks, indicating the corneal swelling is suppressed in the presence of KUS121 or KUS187.
Fig. 8 shows the TUNEL-stained images of tissue sections of rabbit corneas preserved in Optisol-GS for one to four weeks, indicating the apoptosis is suppressed in the presence of KUS121 or KUS187. Images of three different fields are shown for each section.
Fig. 9 shows the iodopropidium (PI)-stained images of the same fields as Fig. 8.
Fig. 10 shows the percentages of the TUNEL-positive cells in tissue sections of rabbit corneas preserved in Optisol-GS for one to four weeks. The percentages were lower in the presence of KUS121 or KUS187 than in the control group through the test period.
Fig. 11 shows the Na+/K+ ATPase-stained images of tissue sections of rabbit corneas preserved in Optisol-GS for one to four weeks, indicating that the corneal endothelial cells were maintained better in the presence of KUS121 or KUS187 than in the control group from week 1.
Fig. 12 shows the PI-stained images of the same fields as Fig. 11.
Fig. 13 shows the transmission electron microscopic images of tissue sections of rabbit corneas preserved in Optisol-GS for one week. Vacuolization of the corneal epithelial cells, loss of the microvilli, and swelling of the corneal stroma and the corneal endothelial cells were observed in the control, but not in the presence of KUS121.

### DETAILED DESCRIPTION

When a numerical value is accompanied with the term "about", the value is intended to represent any value in the range of -10% of the value to +10% of the value. For example, "about 20" means "a value from 18 to 22." A range defined with a value of the lower limit and a value of the upper limit covers all values from the lower limit to the upper limit, including the values of the both limits. When a range is accompanied with the term "about", the both limits are read as accompanied with the term. For example, "about 20 to 30" is read as "18 to 33."

Unless otherwise defined, the terms used herein are read as generally understood by a skilled person in the technical fields such as organic chemistry, medical sciences, pharmaceutical sciences, molecular biology, and microbiology. Several terms used herein are defined as described below. The definitions herein take precedence over the general understanding.

"Alkyl" refers to a monovalent saturated aliphatic hydrocarbyl group having 1 to 10 carbon atoms, preferably 1 to 6 carbon atoms. Examples of the alkyl include, but are not limited to, linear and branched hydrocarbyl groups, such as methyl, ethyl, n-propyl, isopropyl, n-butyl, isobutyl, secbutyl, t-butyl, n-pentyl, and neopentyl.

The wording "substituted" as a word qualifying a name of a group means that one or more hydrogen atoms of the group are, identically or differently, replaced by one or more substituents defined herein.

"Alkylene" refers to a divalent saturated aliphatic hydrocarbyl group having 1 to 10 carbon atoms, preferably 1 to 6 carbon atoms. Alkylene groups include branched and straight chain hydrocarbyl groups.

"Alkoxy" refers to the group -O-alkyl in which alkyl is as defined herein. Examples of the alkoxy include, but are not limited to, methoxy, ethoxy, n-propoxy, isopropoxy, n-butoxy, t-butoxy, sec-butoxy, and n-pentoxy.

"Aryl" refers to a monovalent aromatic carbocyclic group of 6 to 14 carbon atoms having a single ring (e.g., phenyl) or multiple condensed rings (e.g., naphthyl or anthryl). Typical aryl groups include phenyl and naphthyl.

"Aryloxy" refers to the group -O-aryl, in which aryl is as defined herein. Examples of the aryloxy include phenoxy and naphthoxy.

"Cyano" refers to the group -CN.

"Carboxyl" or "carboxy" refers to the group -COOH or a salt thereof.

"Carboxy ester" refers to the group -C(O)O-alkyl in which alkyl is as defined herein.

"Halo" refers to a halogen, especially fluoro, chloro, bromo, or iodo.

"Hydroxy" refers to the group -OH.

A substituent that is not explicitly defined herein is named by describing the name of the terminal functional group of the substituent first and sequentially describing the adjacent functional group toward the point binding to the rest of the compound, unless otherwise indicated. For example, the substituent "arylalkyloxycarbonyl" refers to (aryl)-(alkyl)-O-C(O)-.

Some compounds of formula (I) have enantiomers or diastereomers, depending on their substituents. They may be racemic mixtures or stereoisomerically pure components separated by a known method. Some compounds may be tautomers.

The term "ester" refers to an ester that hydrolyzes in vivo, which may break down readily in a human body to leave the parent compound or a salt thereof. Suitable esters include, for example, those derived from pharmaceutically acceptable aliphatic carboxylic acids, especially alkanoic, alkenoic, cycloalkanoic, and alkanedioic acids, in which each alkyl or alkenyl moiety has, e.g., not more than six carbon atoms. Examples of esters include formates, acetates, propionates, butyrates, acrylates, and ethylsuccinates.

The term "oxide" refers to an oxide wherein a nitrogen of a heteroaryl group is oxidized to form N-oxide.

The term "prodrug" refers to a prodrug of a compound which is, within the scope of sound medical judgment, suitable for use in contact with the tissues of humans and animals without an undue adverse effect such as toxicity, irritation, and allergic response, commensurate with a reasonable benefit/risk ratio, and effective for their intended use. A prodrug is rapidly transformed *in vivo,* for example by hydrolysis in blood, to yield a parent compound represented by the formula above. A general discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

The term "pharmaceutically acceptable salt" may be a salt of a compound of formula (I) with an inorganic or organic acid. Preferred salts include salts with inorganic acids, such as hydrochloric acid, hydrobromic acid, phosphoric acid, or sulfuric acid; or salts with organic carboxylic acids or sulfonic acids, such as acetic acid, trifluoroacetic acid, propionic acid, maleic acid, fumaric acid, malic acid, citric acid, tartaric acid, lactic acid, benzoic acid, methanesulfonic acid, ethanesulfonic acid, benzenesulfonic acid, toluenesulfonic acid, naphthalene sulfonic acid, or naphthalene disulfonic acid.

Pharmaceutically acceptable salts also include salts with conventional bases, such as alkali metal salts (e.g., sodium or potassium salt), alkaline earth metal salts (e.g., calcium or magnesium salt), ammonium salts derived from ammonia or organic amines (e.g., diethylamine, triethylamine, ethyldiisopropylamine, procaine, dibenzylamine, N-methylmorpholine, dihydroabiethylamine, methylpiperidine, L-arginine, creatine, choline, L-lysine, ethylenediamine, benzathine, ethanolamine, meglumine, or tromethamine), especially a sodium salt.

The term "solvate" means a compound of formula (I) in a form of a complex formed by coordination with a solvent molecule in a solid or liquid state. A suitable solvate is a hydrate.

The term "compound of formula (I)" referred in the disclosure is intended to include its esters, oxides, prodrugs, pharmaceutically acceptable salts, and solvates, unless this is inadequate in the context.

In an embodiment, each Ra radical in formula (I) is independently selected from the group consisting of halo, hydroxy, alkyl, halo-substituted alkyl, and alkoxy.

In an embodiment, each Ra radical in formula (I) is independently selected from the group consisting of halo and alkyl.

In an embodiment, formula (I) has two Ra radicals which are halo and alkyl.

In an embodiment, each Ra radical in formula (I) is independently selected from the group consisting of hydroxy, alkyl, and alkoxy.

In an embodiment, formula (I) has three Ra radicals which are hydroxy, alkyl, and alkoxy.

In an embodiment, the compound of formula (I) is selected from Compounds 1 to 53 listed in Table 1 below:

**[Table 1-1]**

| No. | Compound Name |
|---|---|
| 1 | 4-amino-3-(6-phenylpyridine-3-ylazo)naphthalene-1-sulfonic acid |
| 2 | 4-amino-3-(6-p-tolylpyridine-3-ylazo)naphthalene-1-sulfoni c acid |
| 3 | 4-amino-3-(6-m-tolylpyridine-3-ylazo)naphthalene-1-sulfoni c acid |
| 4 | 4-amino-3-(6-o-tolylpyridine-3-ylazo)naphthalene-1-sulfoni c acid |
| 5 | 4-amino-3-(6-biphenyl-2-ylpyridine-3-ylazo)naphthalene-1-s ulfonic acid |
| 6 | 3-[6-(2-acetylphenyl)pyridine-3-ylazo]-4-aminonaphthalene-1-sulfonic acid |
| 7 | 3-[6-(3-acetylphenyl)pyridine-3-ylazo]-4-aminonaphthalene-1-sulfonic acid |
| 8 | 3-[6-(4-acetylphenyl)pyridine-3-ylazo]-4-aminonaphthalenes ulfonic acid |
| 9 | 4-amino-3-[6-(2,4-dichlorophenyl)pyridine-3-ylazo]naphthal ene-1-sulfonic acid |
| 10 | 4-amino-3-[6-(2-trifluoromethylphenyl)pyridine-3-ylazo]nap hthalene-1-sulfonic acid |
| 11 | 4-amino-3-[6-(4-trifluoromethylphenyl)pyridine-3-ylazo]nap hthalene-1-sulfonic acid |
| 12 | 4-amino-3-[6-(2-chlorophenyl)pyridine-3-ylazo]naphthalene-1-sulfonic acid |
| 13 | 4-amino-3-[6-(3-chlorophenyl)pyridine-3-ylazo]naphthalene-1-sulfonic acid |
| 14 | 4-amino-3-[6-(4-chlorophenyl)pyridine-3-ylazo]naphthalene-1-sulfonic acid |
| 15 | 4-amino-3-[6-(2-methoxyphenyl)pyridine-3-ylazo]naphthalene -1-sulfonic acid |
| 16 | 4-amino-3-[6-(4-methoxyphenyl)pyridine-3-ylazo]naphthalene -1-sulfonic acid |
| 17 | 4-amino-3-[6-(2-isopropoxyphenyl)pyridine-3-ylazo]naphthal ene-1-sulfonic acid |
| 18 | 4-amino-3-[6-(4-isopropoxyphenyl)pyridine-3-ylazo]naphthal ene-1-sulfonic acid |
| 19 | 4-amino-3-[6-(2-phenoxyphenyl)pyridine-3-ylazo]naphthalene -1-sulfonic acid |
| 20 | 4-amino-3-[6-(3-methoxyphenyl)pyridine-3-ylazo]naphthalene -1-sulfonic acid |

**[Table 1-2]**

| | |
|---|---|
| 21 | 4-amino-3-[6-(2,3-dimethylphenyl)pyridine-3-ylazo]naphthal ene-1-sulfonic acid |
| 22 | 4-amino-3-[6-(2,5-dimethylphenyl)pyridine-3-ylazo]naphthal ene-1-sulfonic acid |
| 23 | 4-amino-3-[6-(3,5-dimethylphenyl)pyridine-3-ylazo]naphthal ene-1-sulfonic acid |
| 24 | 4-amino-3-[6-(3-trifluoromethylphenyl)pyridine-3-ylazo]nap hthalene-1-sulfonic acid |
| 25 | 4-{4-[5-(1-amino-4-sulfonaphthalene-2-ylazo) pyridine-2-yl ]phenyl}-4-oxobutyric acid |
| 26 | 4-amino-3-(6-biphenyl-3-ylpyridine-3-ylazo)naphthalene-1-s ulfonic acid |
| 27 | 4-amino-3-[6-(3-cyanophenyl)pyridine-3-ylazo]naphthalene-1 -sulfonic acid |
| 28 | 4-amino-3-[6-(4-cyanophenyl)pyridine-3-ylazo]naphthalene-1 -sulfonic acid |
| 29 | 4-amino-3-[6-(3,5-bistrifluoromethylphenyl)pyridine-3-ylaz o]naphthalenesulfonic acid |
| 30 | 4-amino-3-[6-(4-benzoylphenyl)pyridine-3-ylazo]naphthalene -1-sulfonic acid |
| 31 | 4-amino-3- [6- (2-propoxyphenyl)pyridine-3-ylazo] naphthalen e-1-sulfonic acid |
| 32 | 4-amino-3-[6-(4-fluoro-2-methylphenyl)pyridine-3-ylazo]nap hthalene-1-sulfonic acid |
| 33 | 4-amino-3-[6-(5-fluoro-2-propoxyphenyl)pyridine-3-ylazo]na phthalene-1-sulfonic acid |
| 34 | 4-amino-3-[6-(2-fluoro-6-propoxyphenyl)pyridine-3-ylazo]na phthalene-1-sulfonic acid |
| 35 | 4-amino-3-[6-(4-fluoro-2-propoxyphenyl)pyridine-3-ylazo]na phthalene-1-sulfonic acid |
| 36 | 4-amino-3-[6-(5-fluoro-2-methylphenyl)pyridine-3-ylazo]nap hthalene-1-sulfonic acid |
| 37 | 4-amino-3-[6-(2-fluoro-5-methylphenyl)pyridine-3-ylazo] nap hthalene-1-sulfonic acid |
| 38 | 4-amino-3-[6-(2-butoxyphenyl)pyridine-3-ylazo]naphthalene-1-sulfonic acid |
| 39 | 4-amino-3-[6-(2-hexyloxyphenyl)pyridine-3-ylazo]naphthalen e-1-sulfonic acid |
| 40 | 4-amino-3-[6-(4-butylphenyl)pyridine-3-ylazo]naphthalene-1 -sulfonic acid |

**[Table 1-3]**

| | |
|---|---|
| 41 | 4-amino-3-[6-(2-hydroxyphenyl)pyridine-3-ylazo]naphthalene -1-sulfonic acid |
| 42 | 4-amino-3-{6-[2-(6-hydroxyhexyloxy)phenyl]pyridine-3-ylazo }naphthalene-1-sulfonic acid |
| 43 | 4-{2-[5-(1-amino-4-sulfonaphthalene-2-ylazo)pyridine-2-yl] phenoxy}butyric acid |
| 44 | 4-amino-3-{6-[2-(3-hydroxypropoxy)phenyl]pyridine-3-ylazo} naphthalene-1-sulfonic acid |
| 45 | 4-amino-3-[6-(2-isobutoxyphenyl)pyridine-3-ylazo]naphthale ne-1-sulfonic acid |
| 46 | 4-amino-3-[6-(5-chloro-2-hydroxyphenyl)pyridine-3-ylazo]na phthalene-1-sulfonic acid |
| 47 | 4-amino-3-[6-(4-methylbiphenyl-2-yl)pyridine-3-ylazo]napht halene-1-sulfonic acid |
| 48 | 4-amino-3-[6-(4'-chloro-4-methylbiphenyl-2-yl)pyridine-3-y lazo]naphthalene-1-sulfonic acid |
| 49 | 4-amino-3-[6-(4,3',5'-trimethylbiphenyl-2-yl)pyridine-3-yl azo]naphthalene-1-sulfonic acid |
| 50 | 4-amino-3-[6-(3'-chloro-4-methylbiphenyl-2-yl)pyridine-3-y lazo]naphthalene-1-sulfonic acid |
| 51 | 4-amino-3-[6-(2,6-dimethylphenyl)pyridine-3-ylazo]naphthal ene-1-sulfonic acid |
| 52 | 4-amino-3-[6-(3-formyl-2-isopropoxy-5-methylphenyl)pyridin e-3-ylazo]naphthalene-1-sulfonic acid |
| 53 | 4-amino-3-[6-(3-formyl-2-butoxy-5-methylphenyl)pyridine-3-ylazo]naphthalene-1-sulfonic acid |

In an embodiment, preferred is 4-amino-3-[6-(4-fluoro-2-methylphenyl)pyridine-3-ylazo]naphthalene-1-sulfonic acid, which is represented by the following formula: 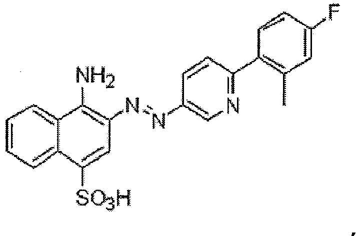 or an ester, oxide, prodrug, pharmaceutically acceptable salt or solvate thereof, especially a sodium salt.

In an embodiment, preferred is 4-amino-3-[6-(3-formyl-2-butoxy-5-methylphenyl)pyridine-3-ylazo]naphthalene-1-sulfonic acid, which is represented by the following formula: 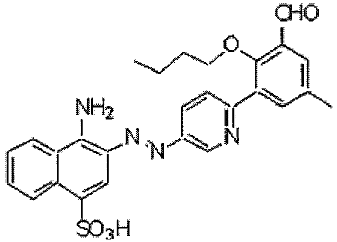 or an ester, oxide, prodrug, pharmaceutically acceptable salt or solvate thereof, especially a sodium salt.

The characteristics of the compounds of formula (I), especially the compounds listed above, and the methods for synthesizing them are described in WO2012/014994, WO2012/043891, WO2014/129495, and WO2015/129809 (Patent Literatures 1 to 4) in detail.

As demonstrated by the Examples below, the compounds of formula (I) protect corneal endothelial cells and corneal epithelial cells and suppress cell death, and thus can protect a cornea. The term "protecting a cornea" or "corneal protection" as used herein means inhibiting cell death of corneal endothelial cells and corneal epithelial cells, inhibiting corneal abnormality (e.g., edema, swelling, or turbidity), and/or inhibiting the progression of corneal abnormality, wherein the cornea is a cornea present in the subject's eye or a corneal graft excised for transplantation. The subject may or may not suffer from a corneal disease.

Accordingly, an aspect of the disclosure provides a composition for protecting a cornea comprising a compound of formula (I).

An aspect of the disclosure provides a method for protecting a cornea comprising administering a compound of formula (I) to a subject in need thereof.

An aspect of the disclosure provides a method for protecting a cornea comprising allowing the cornea to contact with a compound of formula (I).

An aspect of the disclosure provides a compound of formula (I) for use in protecting a cornea.

An aspect of the disclosure provides use of a compound of formula (I) for protecting a cornea.

An aspect of the disclosure provides use of a compound of formula (I) for manufacturing a composition for protecting a cornea.

In virtue of the corneal protection effect, the compounds of formula (I) are useful, for example, in a composition for treating or preventing a corneal disease, a composition for ocular irrigation, or a composition for preserving a corneal graft.

### (1) Pharmaceutical composition for treating or preventing a corneal disease

The term "corneal disease" refers to a disease accompanied by a corneal lesion, disorder, or damage. The corneal disease may be a corneal endothelial disease or a corneal epithelial disease. Examples of the corneal endothelial diseases include diseases affecting cornea endothelial cells, such as corneal endothelial degenerations (e.g., cornea guttata, Fuchs endothelial corneal dystrophy, posterior polymorphous corneal dystrophy, iridocorneal endothelial syndrome, and congenital hereditary corneal endothelial dystrophy), viral diseases (e.g., cytomegalovirus endotheliitis and herpetic endotheliitis), exfoliation syndrome, and corneal endothelial graft rejection; as well as inflammation or physical damage associated with external factors, such as keratouveitis, interstitial keratitis, corneal endotheliitis, corneal endothelial cell loss after corneal transplantation, corneal injury after intraocular surgery (e.g., cataract surgery, vitreous surgery, glaucoma surgery), corneal injury induced by glaucomatous attack, corneal injury caused by long-term contact lens use, corneal trauma, intrapartum corneal trauma, and bullous keratopathy. Examples of the corneal epithelial diseases include superficial punctate keratitis, corneal erosion, corneal ulcer, dry eye, keratoconjunctivitis sicca, corneal marginal ulcer, and corneal trauma.

The term "treating" or "treatment" as used herein means that in a subject suffering from a corneal disease a cause of the corneal disease is reduced or removed, progression of the corneal disease is delayed or stopped, and/or a symptom of the corneal disease is reduced, alleviated, ameliorated, or removed.

The term "preventing" or "prevention" as used herein means that in a subject, especially a subject that is susceptible to a corneal disease but has not been affected with the disease yet, the disease onset is prevented or the possibility of the disease onset is decreased. Examples of the subjects that are susceptible to a corneal disease but have not been affected with the disease yet include subjects who have genetic predisposition to a corneal disease, such as Fuchs endothelial corneal dystrophy and posterior polymorphous corneal dystrophy, and subjects who have a causal factor of a corneal disease. Examples of the causal factors include intraocular surgery (e.g., cataract surgery, vitreous surgery, glaucoma surgery), corneal transplantation, laser therapy, rapid increase of intraocular pressure, inflammation, trauma, and conjunctivitis.

The subject who receives treatment or prevention of a corneal disease may be an animal, typically a mammal (e.g., a human, mouse, rat, hamster, rabbit, cat, dog, bovine, sheep, or monkey), especially a human.

The pharmaceutical composition may be administered in any way through a generally known administration route, for example, by oral administration, parenteral administration, injection, infusion, ocular administration, anterior chamber administration, or intravitreal administration. The composition may be in a dosage form suitable for each administration route.

Dosage forms suitable for oral administration include granules, fine granules, powders, coated tablets, tablets, suppositories, fine powders, capsules, microcapsules, chewable tablets, liquids, suspensions, and emulsions. Dosage forms suitable for injection may be common dosage forms, e.g., those suitable for intravenous injection, infusion, intraocular injection, or extended release of an active ingredient. Dosage forms suitable for ocular administration include liquids, suspensions, and emulsions.

Such dosage forms can be manufactured by formulating an active ingredient by a conventional method. If necessary for the formulation, any one of various pharmaceutically acceptable excipients may be added. Any excipient may be used in accordance with the employed dosage form. Examples of the excipients include buffering agents, surfactants, stabilizers, preservatives, fillers, diluents, additives, disintegrants, binders, coating agents, lubricants, lubricating agents, flavoring agents, sweeteners, and solubilizers.

In an embodiment, the pharmaceutical composition is an eye drop. Eye drops typically may be prepared by using an isotonizing agent such as sodium chloride or concentrated glycerin; a buffering agent such as sodium phosphate or sodium acetate; a surfactant such as polyoxyethylene sorbitan monooleate, polyoxyl stearate 40, or polyoxyethylene hardened castor oil; a stabilizer such as sodium citrate or sodium edetate; and a preservative such as benzalkonium chloride or paraben, but components of eye drops are not limited thereto. Eye drops may have pH within a range accepted for ophthalmic formulations, for example, within the range of pH 4 to 8.

The pharmaceutical composition may be an ocular irrigation solution or a composition to be added to an ocular irrigation solution. The pharmaceutical composition may be a corneal preservation solution or a composition to be added to a corneal preservation solution. The details are as described later.

The dosage and the number of doses of the pharmaceutical composition may be appropriately set by those skilled in the art so that an effective amount of a compound of formula (I) is administered to the subject, on the basis of factors such as the animal species of the subject, the health condition of the subject, the age, the weight, the administration route, and the dosage form. Those skilled in the art may easily determine an effective amount in a given situation by routine experimentation within the range of ordinary skill and determination of clinicians. For example, for systemic administration a compound of formula (I) may be administered in the range of about 0.001 to 1,000 mg/kg body weight/day, about 0.01 to 300 mg/kg body weight/day, about 0.1 to 100 mg/kg body weight/day, or about 1.0 to 30 mg/kg body weight/day. For example, a compound of formula (I) may be administered by local administration in the range of about 0.01 to 100 µg/kg body weight/day, about 0.1 to 10 µg/kg body weight/day, or about 0.3 to 3 µg/kg body weight/day. A compound of formula (I) may be administered once a day or more, for example, two, three, or four times a day.

A compound of formula (I) may be used alone or in combination with at least one other active ingredient, especially an active ingredient for treating or preventing a corneal disease. For example, the pharmaceutical composition may comprise at least one active ingredient in addition to a compound of formula (I).

When some ingredients are used "in combination", a dosage form containing all the ingredients may be administered, or a combination of dosage forms containing the ingredients separately may be administered. All ingredients may be simultaneously administered, or any ingredient may be administered at later time point, as long as the ingredients are used for preventing and/or treating a corneal disease. Two or more further active ingredients may be used in combination with a compound of formula (I). Examples of the active ingredients suitable for use in combination include anti-inflammatory agents, antibacterial agents, antifungal agents, immunosuppressive agents, and antiviral agents.

An ophthalmic therapy other than drug therapy may be combined with treatment or prevention of a corneal disease with a compound of formula (I). Examples of the suitable therapies include surgery, photodynamic therapy, gene therapy, regenerative therapy, corneal implantation, and laser therapy.

An aspect of the disclosure provides a method for treating or preventing a corneal disease comprising administering a compound of formula (I) to a subject in need thereof.

An aspect of the disclosure provides a compound of formula (I) for use in treating or preventing a corneal disease.

An aspect of the disclosure provides use of a compound of formula (I) for treating or preventing a corneal disease.

An aspect of the disclosure provides use of a compound of formula (I) for manufacturing a composition for treating or preventing a corneal disease.

### (2) Composition for ocular irrigation

Ocular surgery, such as cataract surgery, vitreous surgery, or glaucoma surgery, may cause a corneal disease such as corneal edema or corneal turbidity. Addition of a compound of formula (I) to an ocular irrigation solution, which is usually used in ocular surgery for ocular irrigation and washing, may protect a cornea and prevent a corneal disease.

The composition for ocular irrigation may be in the form of solid, semi-solid, or liquid. The composition may be provided as a ready-to-use ocular irrigation solution or a composition to be added to an ocular irrigation solution. When the composition is provided as an ocular irrigation solution, other components contained in the composition may be equivalent to those of existing ocular irrigation solutions and preferably components similar to those of aqueous humor, and may contain a further component such as a glutathione, e.g., oxyglutathione, and an antibiotic. Examples of the existing ocular irrigation solutions include, but are not limited to, physiological saline, Ringer's lactate solution, BSS PLUS (trade name), Opeguard (trade name), and oxyglutathione ocular irrigating solution (trade name). When the composition is provided as a composition to be added to an ocular irrigation solution, the composition may contain any component unless it impairs the function and safety of the ocular irrigation solution.

The composition for ocular irrigation may contain any amount of a compound of formula (I) as long as the compound is present at an effective concentration when the composition is used for ocular irrigation. The effective concentration of the compound of formula (I) in use for ocular irrigation may be, for example, in the range of 0.1 µM to 10 mM, 1 µM to 1 mM, 2 µM to 200 µM, 5 µM to 100 µM, or 10 µM to 100 µM.

An aspect of the disclosure provides a method for performing ocular irrigation in a subject undergoing ocular surgery comprising using an ocular irrigation solution comprising a compound of formula (I).

An aspect of the disclosure provides a method for protecting a cornea comprising performing ocular irrigation in a subject undergoing ocular surgery with an ocular irrigation solution comprising a compound of formula (I).

An aspect of the disclosure provides a compound of formula (I) for use in ocular irrigation.

An aspect of the disclosure provides a compound of formula (I) for use in protecting a cornea in ocular irrigation.

An aspect of the disclosure provides use of a compound of formula (I) for ocular irrigation.

An aspect of the disclosure provides use of a compound of formula (I) for protecting a cornea in ocular irrigation.

An aspect of the disclosure provides use of a compound of formula (I) for manufacturing a composition for ocular irrigation.

An aspect of the disclosure provides use of a compound of formula (I) for manufacturing a composition for protecting a cornea in ocular irrigation.

### (3) Composition for preserving a corneal graft

In corneal transplantation a cornea donated from a corneal donor has to be preserved until transplantation surgery. Addition of a compound of formula (I) to a corneal preservation solution may protect and preserve a cornea in better condition.

The composition for preserving a corneal graft may be in the form of solid, semi-solid, or liquid. The composition may be provided as a ready-to-use corneal preservation solution or a composition to be added to a corneal preservation solution. When the composition is provided as a corneal preservation solution, other components contained in the composition may be equivalent to those of existing corneal preservation solutions. Examples of such components include basal media, buffering agents, chondroitin sulfate, dextran, vitamins, ATP precursors, and antibiotics. Examples of the existing corneal preservation solutions include, but are not limited to, M199 medium, MEMα medium, MK medium, CSM medium, CSMD medium, DMEM/F-12 medium, Optisol-GS (trade name), K-Sol (trade name), and EP2 (trade name). When the composition is provided as a composition to be added to a corneal preservation solution, the composition may contain any component unless it impairs the function and safety of the corneal preservation solution.

The composition for preserving a corneal graft may contain any amount of a compound of formula (I) as long as the compound is present at an effective concentration when the composition is used for preserving a corneal graft. The effective concentration of the compound of formula (I) in use for preserving a corneal graft may be, for example, in the range of 0.1 µM to 10 mM, 1 µM to 1 mM, 2 µM to 200 µM, 5 µM to 100 µM, or 10 µM to 100 µM.

An aspect of the disclosure provides a method for preserving a corneal graft comprising preserving a corneal graft in a corneal preservation solution comprising a compound of formula (I).

An aspect of the disclosure provides a method for protecting a corneal graft comprising preserving the corneal graft in a corneal preservation solution comprising a compound of formula (I).

An aspect of the disclosure provides a compound of formula (I) for use in preserving a corneal graft.

An aspect of the disclosure provides a compound of formula (I) for use in protecting a cornea in preserving a corneal graft.

An aspect of the disclosure provides use of a compound of formula (I) for preserving a corneal graft.

An aspect of the disclosure provides use of a compound of formula (I) for protecting cornea in preserving a corneal graft.

An aspect of the disclosure provides use of a compound of formula (I) for manufacturing a composition for preserving a corneal graft.

An aspect of the disclosure provides use of a compound of formula (I) for manufacturing a composition for protecting a cornea in preserving a corneal graft.

For example, the disclosure provides the following embodiments.
[1] A composition for protecting a cornea, comprising a compound of formula (I): 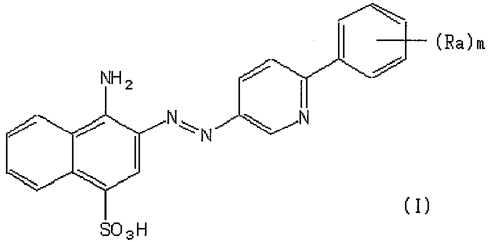 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, pharmaceutically acceptable salt or solvate thereof.
[2] The composition according to item 1, wherein each Ra radical is independently selected from the group consisting of halo, hydroxy, alkyl, halo-substituted alkyl, and alkoxy.
[3] The composition according to item 1 or 2, wherein each Ra radical is independently selected from the group consisting of halo and alkyl.
[4] The composition according to any one of items 1 to 3, wherein formula (I) has two Ra radicals which are halo and alkyl.
[5] The composition according to item 1 or 2, wherein each Ra radical is independently selected from the group consisting of hydroxy, alkyl, and alkoxy.
[6] The composition according to item 1, 2, or 5, wherein formula (I) has three Ra radicals which are hydroxy, alkyl, and alkoxy.
[7] The composition according to item 1, wherein the compound of formula (I) is selected from the compounds listed in Table 1.
[8] The composition according to item 1, 2, or 7, wherein the compound of formula (I) is 4-amino-3-[6-(4-fluoro-2-methylphenyl)pyridine-3-ylazo]naphthalene-1-sulfonic acid or 4-amino-3-[6-(3-formyl-2-butoxy-5-methylphenyl)pyridine-3-ylazo]naphthalene-1-sulfonic acid.
[9] The composition according to any one of items 1 to 4, 7, and 8, wherein the compound of formula (I) is 4-amino-3-[6-(4-fluoro-2-methylphenyl)pyridine-3-ylazo]naphthalene-1-sulfonic acid.
[10] The composition according to any one of items 1 to 4 and 7 to 9, comprising 4-amino-3-[6-(4-fluoro-2-methylphenyl)pyridine-3-ylazo]naphthalene-1-sulfonic acid sodium salt.
[11] The composition according to any one of items 1, 2, and 5 to 8, wherein the compound of formula (I) is 4-amino-3-[6-(3-formyl-2-butoxy-5-methylphenyl)pyridine-3-ylazo]naphthalene-1-sulfonic acid.
[12] The composition according to any one of items 1, 2, 5 to 8, and 11, comprising 4-amino-3-[6-(3-formyl-2-butoxy-5-methylphenyl)pyridine-3-ylazo]naphthalene-1-sulfonic acid sodium salt.
[13] The composition according to any one of items 1 to 12, wherein the composition is for treating or preventing a corneal disease.
[14] The composition according to item 13, wherein the corneal disease is a corneal endothelial disease.
[15] The composition according to item 14, wherein the corneal endothelial disease is cornea guttata, Fuchs endothelial corneal dystrophy, posterior polymorphous corneal dystrophy, iridocorneal endothelial syndrome, congenital hereditary corneal endothelial dystrophy, cytomegalovirus endotheliitis, herpetic endotheliitis, exfoliation syndrome, corneal endothelial graft rejection, keratouveitis, interstitial keratitis, corneal endotheliitis, corneal endothelial cell loss after corneal transplantation, corneal injury after intraocular surgery, corneal injury induced by glaucomatous attack, corneal injury caused by long-term contact lens use, corneal trauma, intrapartum corneal trauma, or bullous keratopathy.
[16] The composition according to item 14 or 15, wherein the corneal endothelial disease is Fuchs endothelial corneal dystrophy or bullous keratopathy.
[17] The composition according to item 13, wherein the corneal disease is a corneal epithelial disease.
[18] The composition according to item 17, wherein the corneal epithelial disease is superficial punctate keratitis, corneal erosion, corneal ulcer, dry eye, keratoconjunctivitis sicca, corneal marginal ulcer, or corneal trauma.
[19] The composition according to item 13, wherein the corneal disease is cornea guttata, Fuchs endothelial corneal dystrophy, posterior polymorphous corneal dystrophy, iridocorneal endothelial syndrome, congenital hereditary corneal endothelial dystrophy, cytomegalovirus endotheliitis, herpetic endotheliitis, exfoliation syndrome, corneal endothelial graft rejection, keratouveitis, interstitial keratitis, corneal endotheliitis, corneal endothelial cell loss after corneal transplantation, corneal injury after intraocular surgery, corneal injury induced by glaucomatous attack, corneal injury caused by long-term contact lens use, corneal trauma, intrapartum corneal trauma, bullous keratopathy, superficial punctate keratitis, corneal erosion, dry eye, keratoconjunctivitis sicca, or corneal marginal ulcer.
[20] The composition according to item 13 or 19, wherein the corneal disease is Fuchs endothelial corneal dystrophy, bullous keratopathy, corneal erosion, dry eye, or corneal trauma.
[21] The composition according to any one of items 1 to 20, wherein the composition is an eye drop.
[22] The composition according to any one of items 1 to 20, wherein the composition is an ocular irrigation solution or a composition to be added to an ocular irrigation solution.
[23] The composition according to any one of items 1 to 20, wherein the composition is a solution for preserving a corneal graft or a composition to be added to a solution for preserving a corneal graft.
[24] A pharmaceutical composition for treating or preventing a corneal disease, comprising a compound of formula (I): 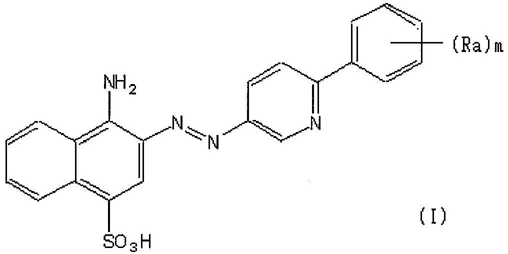 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, pharmaceutically acceptable salt or solvate thereof.
[25] The composition according to item 24, wherein each Ra radical is independently selected from the group consisting of halo, hydroxy, alkyl, halo-substituted alkyl, and alkoxy.
[26] The composition according to item 24 or 25, wherein each Ra radical is independently selected from the group consisting of halo and alkyl.
[27] The composition according to any one of items 24 to 26, wherein formula (I) has two Ra radicals which are halo and alkyl.
[28] The composition according to item 24 or 25, wherein each Ra radical is independently selected from the group consisting of hydroxy, alkyl, and alkoxy.
[29] The composition according to item 24, 25, or 28, wherein formula (I) has three Ra radicals which are hydroxy, alkyl, and alkoxy.
[30] The composition according to item 24, wherein the compound of formula (I) is selected from the compounds listed in Table 1.
[31] The composition according to item 24, 25, or 30, wherein the compound of formula (I) is 4-amino-3-[6-(4-fluoro-2-methylphenyl)pyridine-3-ylazo]naphthalene-1-sulfonic acid or 4-amino-3-[6-(3-formyl-2-butoxy-5-methylphenyl)pyridine-3-ylazo]naphthalene-1-sulfonic acid.
[32] The composition according to any one of items 24 to 27, 30, and 31, wherein the compound of formula (I) is 4-amino-3-[6-(4-fluoro-2-methylphenyl)pyridine-3-ylazo]naphthalene-1-sulfonic acid.
[33] The composition according to any one of items 24 to 27 and 30 to 32, comprising 4-amino-3-[6-(4-fluoro-2-methylphenyl)pyridine-3-ylazo]naphthalene-1-sulfonic acid sodium salt.
[34] The composition according to any one of items 24, 25, and 28 to 31, wherein the compound of formula (I) is 4-amino-3-[6-(3-formyl-2-butoxy-5-methylphenyl)pyridine-3-ylazo]naphthalene-1-sulfonic acid.
[35] The composition according to any one of items 24, 25, 28 to 31, and 34, comprising 4-amino-3-[6-(3-formyl-2-butoxy-5-methylphenyl)pyridine-3-ylazo]naphthalene-1-sulfonic acid sodium salt.
[36] The composition according to any one of items 24 to 35, wherein the corneal disease is a corneal endothelial disease.
[37] The composition according to item 36, wherein the corneal endothelial disease is cornea guttata, Fuchs endothelial corneal dystrophy, posterior polymorphous corneal dystrophy, iridocorneal endothelial syndrome, congenital hereditary corneal endothelial dystrophy, cytomegalovirus endotheliitis, herpetic endotheliitis, exfoliation syndrome, corneal endothelial graft rejection, keratouveitis, interstitial keratitis, corneal injury after cataract surgery, corneal endothelial cell loss after corneal transplantation, corneal injury after retinal surgery, corneal injury after vitreous surgery, corneal injury after glaucoma surgery, corneal injury induced by glaucomatous attack, corneal injury caused by long-term contact lens use, corneal trauma, intrapartum corneal trauma, or bullous keratopathy.
[38] The composition according to item 36 or 37, wherein the corneal endothelial disease is Fuchs endothelial corneal dystrophy or bullous keratopathy.
[39] The composition according to any one of items 24 to 35, wherein the corneal disease is a corneal epithelial disease.
[40] The composition according to item 39, wherein the corneal epithelial disease is corneal erosion, dry eye, or corneal trauma.
[41] The composition according to any one of items 24 to 35, wherein the corneal disease is cornea guttata, Fuchs endothelial corneal dystrophy, posterior polymorphous corneal dystrophy, iridocorneal endothelial syndrome, congenital hereditary corneal endothelial dystrophy, cytomegalovirus endotheliitis, herpetic endotheliitis, exfoliation syndrome, corneal endothelial graft rejection, keratouveitis, interstitial keratitis, corneal injury after cataract surgery, corneal endothelial cell loss after corneal transplantation, corneal injury after retinal surgery, corneal injury after vitreous surgery, corneal injury after glaucoma surgery, corneal injury induced by glaucomatous attack, corneal injury caused by long-term contact lens use, corneal trauma, intrapartum corneal trauma, bullous keratopathy, corneal erosion, or dry eye.
[42] The composition according to any one of items 24 to 35 and 41, wherein the corneal disease is Fuchs endothelial corneal dystrophy, bullous keratopathy, corneal erosion, dry eye, or corneal trauma.
[43] The composition according to any one of items 24 to 42, wherein the composition is an eye drop.
[44] The composition according to any one of items 24 to 42, wherein the composition is an ocular irrigation solution or a composition to be added to an ocular irrigation solution.
[45] The composition according to any one of items 24 to 42, wherein the composition is a solution for preserving a corneal graft or a composition to be added to a solution for preserving a corneal graft.
[46] A composition for ocular irrigation, comprising a compound of formula (I): 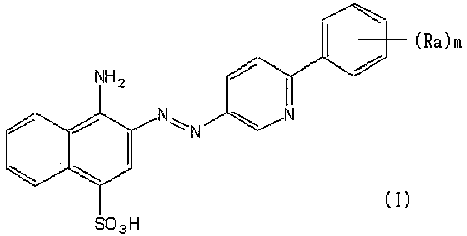 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, pharmaceutically acceptable salt or solvate thereof.
[47] The composition according to item 46, wherein each Ra radical is independently selected from the group consisting of halo, hydroxy, alkyl, halo-substituted alkyl, and alkoxy.
[48] The composition according to item 46 or 47, wherein each Ra radical is independently selected from the group consisting of halo and alkyl.
[49] The composition according to any one of items 46 to 48, wherein formula (I) has two Ra radicals which are halo and alkyl.
[50] The composition according to item 46 or 47, wherein each Ra radical is independently selected from the group consisting of hydroxy, alkyl, and alkoxy.
[51] The composition according to item 46, 47, or 50, wherein formula (I) has three Ra radicals which are hydroxy, alkyl, and alkoxy.
[52] The composition according to item 46, wherein the compound of formula (I) is selected from the compounds listed in Table 1.
[53] The composition according to item 46, 47, or 52, wherein the compound of formula (I) is 4-amino-3-[6-(4-fluoro-2-methylphenyl)pyridine-3-ylazo]naphthalene-1-sulfonic acid or 4-amino-3-[6-(3-formyl-2-butoxy-5-methylphenyl)pyridine-3-ylazo]naphthalene-1-sulfonic acid.
[54] The composition according to any one of items 46 to 49, 52, and 53, wherein the compound of formula (I) is 4-amino-3-[6-(4-fluoro-2-methylphenyl)pyridine-3-ylazo]naphthalene-1-sulfonic acid.
[55] The composition according to any one of items 46 to 49 and 52 to 54, comprising 4-amino-3-[6-(4-fluoro-2-methylphenyl)pyridine-3-ylazo]naphthalene-1-sulfonic acid sodium salt.
[56] The composition according to any one of items 46, 47, and 50 to 53, wherein the compound of formula (I) is 4-amino-3-[6-(3-formyl-2-butoxy-5-methylphenyl)pyridine-3-ylazo]naphthalene-1-sulfonic acid.
[57] The composition according to any one of items 46, 47, 50 to 53, and 56, comprising 4-amino-3-[6-(3-formyl-2-butoxy-5-methylphenyl)pyridine-3-ylazo]naphthalene-1-sulfonic acid sodium salt.
[58] The composition according to any one of items 46 to 57, wherein the composition is an ocular irrigation solution.
[59] The composition according to any one of items 46 to 57, wherein the composition is a composition to be added to an ocular irrigation solution.
[60] A composition for preserving a corneal graft, comprising a compound of formula (I): 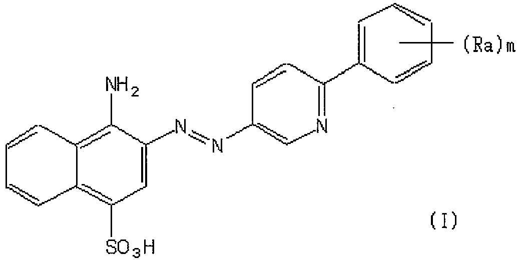 wherein
   Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
   m is an integer selected from 0 to 4,
   or an ester, oxide, pharmaceutically acceptable salt or solvate thereof.
[61] The composition according to item 60, wherein each Ra radical is independently selected from the group consisting of halo, hydroxy, alkyl, halo-substituted alkyl, and alkoxy.
[62] The composition according to item 60 or 61, wherein each Ra radical is independently selected from the group consisting of halo and alkyl.
[63] The composition according to any one of items 60 to 62, wherein formula (I) has two Ra radicals which are halo and alkyl.
[64] The composition according to item 60 or 61, wherein each Ra radical is independently selected from the group consisting of hydroxy, alkyl, and alkoxy.
[65] The composition according to item 60, 61, or 64, wherein formula (I) has three Ra radicals which are hydroxy, alkyl, and alkoxy.
[66] The composition according to item 60, wherein the compound of formula (I) is selected from the compounds listed in Table 1.
[67] The composition according to item 60, 61, or 66, wherein the compound of formula (I) is 4-amino-3-[6-(4-fluoro-2-methylphenyl)pyridine-3-ylazo]naphthalene-1-sulfonic acid or 4-amino-3-[6-(3-formyl-2-butoxy-5-methylphenyl)pyridine-3-ylazo]naphthalene-1-sulfonic acid.
[68] The composition according to any one of items 60 to 63, 66, and 67, wherein the compound of formula (I) is 4-amino-3-[6-(4-fluoro-2-methylphenyl)pyridine-3-ylazo]naphthalene-1-sulfonic acid.
[69] The composition according to any one of items 60 to 63 and 66 to 68, comprising 4-amino-3-[6-(4-fluoro-2-methylphenyl)pyridine-3-ylazo]naphthalene-1-sulfonic acid sodium salt.
[70] The composition according to any one of items 60, 61, and 64 to 67, wherein the compound of formula (I) is 4-amino-3-[6-(3-formyl-2-butoxy-5-methylphenyl)pyridine-3-ylazo]naphthalene-1-sulfonic acid.
[71] The composition according to any one of items 60, 61, 64 to 67 and 70, comprising 4-amino-3-[6-(3-formyl-2-butoxy-5-methylphenyl)pyridine-3-ylazo]naphthalene-1-sulfonic acid sodium salt.
[72] The composition according to any one of items 60 to 71, wherein the composition is a solution for preserving a corneal graft.
[73] The composition according to any one of items 60 to 71, wherein the composition is a composition to be added to a solution for preserving a corneal graft.

### EXAMPLES

In the test examples, the following compounds were employed.
KUS121: 4-amino-3-[6-(4-fluoro-2-methylphenyl)pyridine-3-ylazo]naphthalene-1-sulfonic acid sodium salt
KUS187: 4-amino-3-[6-(3-formyl-2-butoxy-5-methylphenyl)pyridine-3-ylazo]naphthalene-1-sulfonic acid sodium salt
KUS121 and KUS187 were prepared by the method disclosed in WO2012/014994.

### Test 1: Effects of the compounds in cultured rabbit corneal endothelial cells

Whole eyeballs of Japanese white rabbits (Funakoshi Corporation) were cut along corneal limbus with a razor blade and the corneas were isolated. The corneal endothelial cells were mechanically scraped off and collected with a crescent knife. Primary cultures were prepared in a DMEM with 10% fetal bovine serum and 2 ng/ml bFGF. The sub-confluent cells were treated with trypsin and collected, then plated on 12-well plates at the equal density of 4 × 10⁴ cells/well and cultured in the same medium for 24 hours. The cells were subsequently cultured in the following eight different conditions: (1) control (the medium + DMSO), (2) the medium + KUS121 (50 µM), (3) the medium + antimycin (20 µM), (4) the medium + antimycin (20 µM) + KUS121 (50 µM), (5) the medium + oligomycin (10 µM), (6) the medium + oligomycin (10 µM) + KUS121 (50 µM), (7) DMEM with 2 ng/ml bFGF (non-glucose); and (8) DMEM with 2 ng/ml bFGF (non-glucose) + KUS121 (50 µM).

### (1) Cell morphology

After the addition of the agents, sequential phase-contrast images of the cultured corneal endothelial cells were obtained.

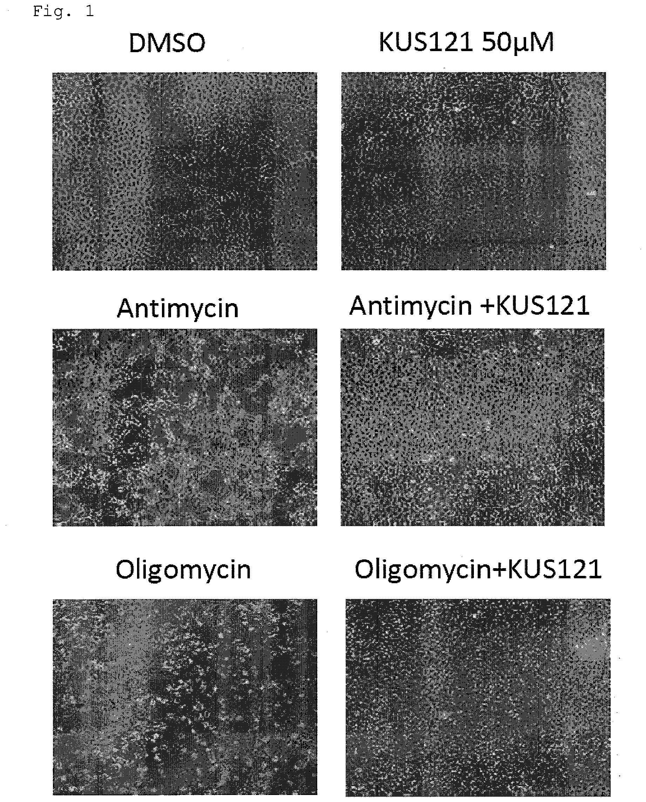

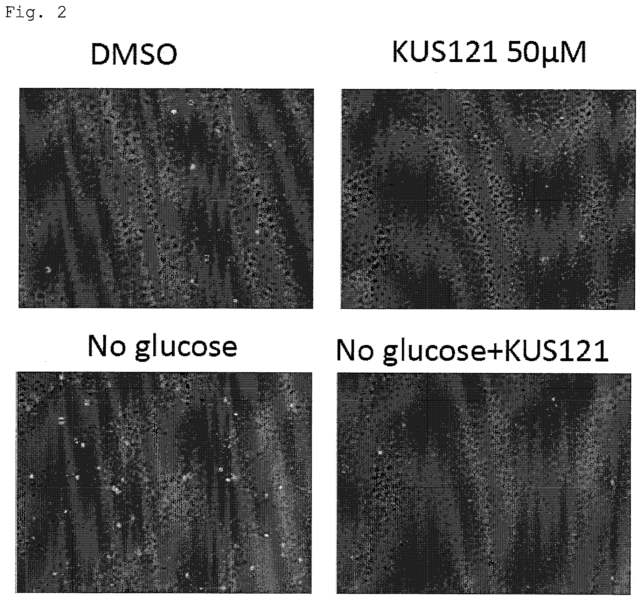

Fig. 1 shows the phase-contrast images at 28 hours after the addition of the agents. The treatment with antimycin or oligomycin caused death of the corneal endothelial cells, but the cell death was suppressed in the presence of KUS121 (50 µM). Fig. 2 shows the phase-contrast images of the cells cultured without glucose at 96 hours after the addition of the agents. The glucose deprivation caused death of the corneal endothelial cells, but the cell death was suppressed in the presence of KUS121 (50 µM).

### (2) Intracellular ATP concentration

The corneal endothelial cells cultured in the presence of the agents (P = 1) were washed with PBS(-), treated with trypsin, suspended in the medium (DMEM with 10% fetal bovine serum and 2 ng/ml bFGF), and counted. Then 2.5 × 10⁴ cells were centrifuged, suspended in PBS(-), and dispensed into 5 wells of a 96-well plate at the density of 5 × 10³ cells/well. The cells were subjected to a luciferase assay ("Cell" ATP Assay reagent, TOYO B-Net CO., LTD.). The fluorescence intensities were measured as relative intracellular ATP concentrations using a luminometer.

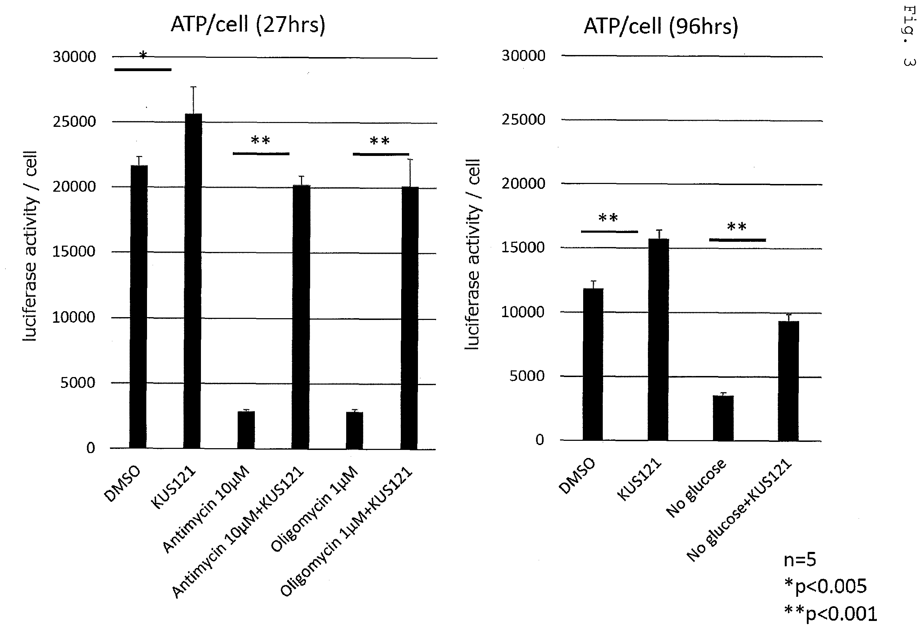

Fig. 3 shows the measurements of the intracellular ATP concentrations. The intracellular ATP concentrations were decreased in the cells treated with antimycin or oligomycin for 27 hours and the cells cultured in the absence of glucose for 96 hours, but the decrease was suppressed in the presence of KUS121.

### Test 2: Effects of the compounds in human corneal endothelial cells from corneas of healthy donors

Corneas for research were imported, from SightLifeTM (Seattle, WA). The cornea endothelial cells were stripped from the corneas with Descemet's membranes and immersed in a corneal preservation solution (Optisol-GS, Bausch & Lomb Incorporated). The cornea endothelial cells were treated with collagenase and collected. The cells were cultured in a conditioned medium which was prepared by culturing 3T3 cells in OptiMEM (Gibco, 3195070) with 10% fetal bovine serum (FBS) for 24 hours and then adding 10 µM SB203580 (Funakoshi, 13067) and 1 µM SB431542 (Wako, 192-16541) to the culture medium.

The corneal endothelial cells were immortalized by the following procedures. SV40 large T antigen and hTERT genes were amplified by PCR, and introduced into a lentiviral vector (pLenti6.3-V5-TOPO; Life Technologies Inc.). The lentiviral vector and three helper plasmids (pLP1, pLP2, pLP/VSVG; Life Technologies Inc.) were transfected into 293T cells (RCB2202; Riken Bioresource Center, Ibaraki, Japan) with a transfection reagent (Fugene HD; Promega Corp., Madison, WI). The culture supernatant containing the virus was collected 48 hours after the transfection and added to the culture medium of the cornea endothelial cells with 5 pg/ml of Polybrene, thereby the SV40 large T antigen and hTERT genes were introduced into the cells. The established immortalized cell line of the normal cornea endothelial cells (iHCECs) was observed by phase-contrast microscopy. The cells had morphology of a monolayer of polygonal cells like the normal corneal endothelial cells. The iHCECs were maintained in Dulbecco's modified Eagle's medium (DMEM) + 10% fetal bovine serum (FBS).

The culture medium was removed from the cultured immortalized human corneal endothelial cells, the cells were washed twice with 1 × PBS(-) warmed to 37°C. Then 1 × PBS(-) was added and the cells were incubated at 37°C (5% CO₂) for 3 minutes. After removal of PBS(-), 0.05% Trypsin-EDTA (NACALAI TESQUE, INC. 322778-34) was added and the cells were incubated at 37°C (5% CO₂) for 5 minutes. The cells were suspended in the following medium and collected by centrifugation at 1500 rpm for 3 minutes.
Medium: DMEM (NACALAI TESQUE, INC. 08456-36) + 10% FBS (Biowest, S1820-500) + 1% P/S (NACALAI TESQUE, INC., 26252-94)

The immortalized human corneal endothelial cells (lot: iHCEC1-1) were plated on 12-well plates at the density of 7 × 10⁴ cells/well and cultured at 37°C (5% CO₂) for 24 hours.
Medium: DMEM + 10% FBS + 1% P/S

The medium was changed to the medium containing KUS121 (10 µM, 25 µM, 50 µM, or 100 µM), and the cells were cultured for 24 hours.
Medium: DMEM + 10% FBS + 1% P/S

The medium was changed to the medium containing 10 µM thapsigargin (WAKO, 209-17281) and KUS121 (10 µM, 25 µM, 50 µM, or 100 µM), and the cells were cultured for 8 hours.
Medium: DMEM + 10% FBS + 1% P/S

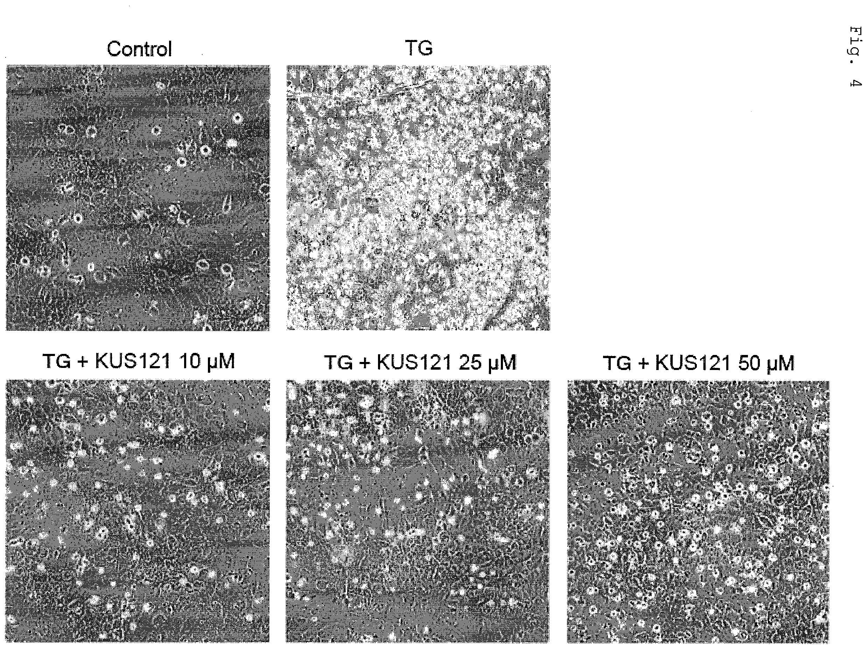

Eight hours after adding thapsigargin the cell morphology and apoptosis were observed by phase-contrast microscopy. The results are shown in Fig. 4. When the immortalized human corneal endothelial cells were treated with thapsigargin (TG) in the absence of KUS121, deformation of the cells, which indicates cell damage, was observed. In the cell groups cultured in the presence of KUS121, the deformation was suppressed. The results suggest that KUS121 decreases the cell damage caused by thapsigargin.

After the observation of the morphology, western blot analysis was performed by the following procedures.

The media were collected from each well on ice, the cells were washed twice with 1 × PBS(-), and the washings were collected. In order to collect suspended cells and dead cells, the collected media and washings were centrifuged at 4°C, 800 g, for 12 minutes, the supernatants were discarded and the pellets were obtained. Protein extraction buffers (RIPA; 50 mM Tris-HCl (pH 7.4), 150 mM NaCl, 1 mM EDTA, 0.1% SDS, 0.5% DOC, 1% NP-40) were added to the washed cells on ice. The cells were suspended together with the pellets of the suspended cells and dead cells. The collected suspensions were sonicated three times for 30 seconds in cold water by sonicator (BIORUPTOR, TOSHO DENKI) and centrifuged at 4°C, 15000 rpm for 10 minutes to collect the supernatants containing proteins.

The protein extracts (7 µg each) were separated by SDS-PAGE and transferred to a nitrocellulose membrane. The following primary antibodies were used; rabbit anti-caspase 3 antibody (Cell Signaling, 9662), rabbit anti-PARP antibody (Cell Signaling, 9542), and mouse anti-GAPDH antibody (MEDICAL & BIOLOGICAL LABORATORIES CO., LTD., M171-3). The secondary antibodies were peroxidase-labeled anti-rabbit and anti-mouse antibodies (GE Healthcare Biosciences, NA931V, NA934V). The primary antibodies, rabbit anti-caspase 3, rabbit anti-PARP antibody, and mouse anti-GAPDH antibody, were diluted at 1:1000, 1:1000, and 1:3000, respectively. The secondary antibodies were diluted at 1:5000. The signals were detected by Chemi Lumi ONE Ultra (NACALAI TESQUE, INC., 11644-40). The intensities of the detected bands were analyzed by luminescent image analyzer LAS-4000 mini (Fujifilm Corporation) and ImageQuantTM software (GE Healthcare).

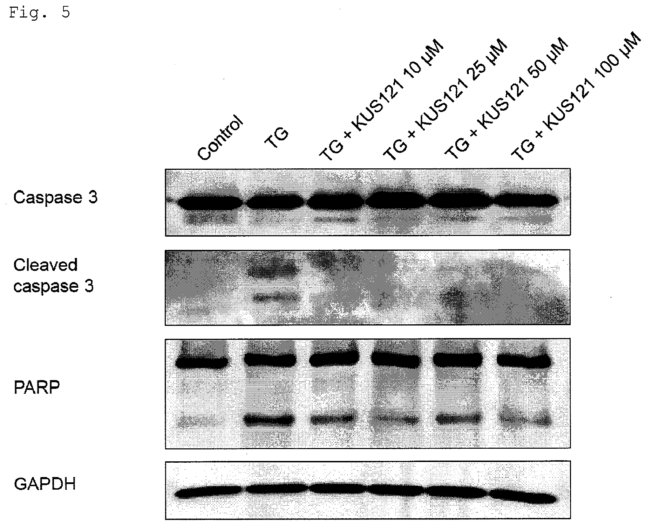

The results are shown in Fig. 5. When the immortalized human corneal endothelial cells were treated with thapsigargin in the absence of KUS121, a band of cleaved caspase 3 (about 17 kDa), the active form of caspase 3, was detected. On the other hand, the cleaved caspase 3 is hardly detected in the presence of KUS121. Thus, the western blot analysis revealed that KUS121 suppresses activation of caspase induced by thapsigargin. The results suggest that KUS121 suppresses cell damage due to ER stress induced by thapsigargin.

### Test 3: Effects of the compounds in rabbit corneas

Japanese white rabbits were euthanized, whole eyeballs were excised, and sclerocorneal preparations were prepared by using a razor blade. Each four sclerocorneal preparations were preserved in the following preservation solutions at 4°C for 4 weeks; (1) Optisol-GS (control), (2) Optisol-GS + KUS121 (50 µM), or (3) Optisol-GS + KUS187 (20 µM). The tissues were sequentially embedded and sectioned.

### (1) Microscopic findings and central corneal thickness

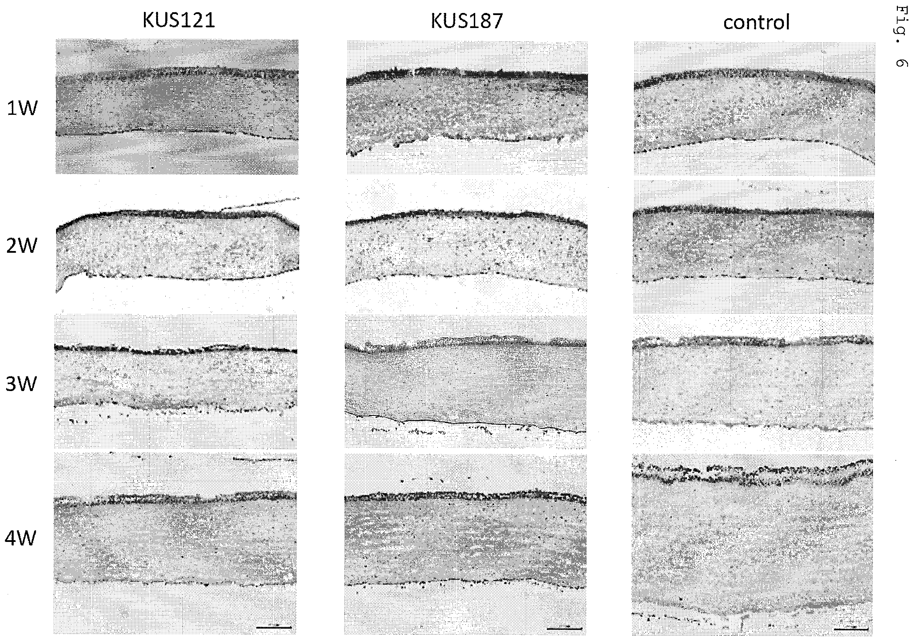

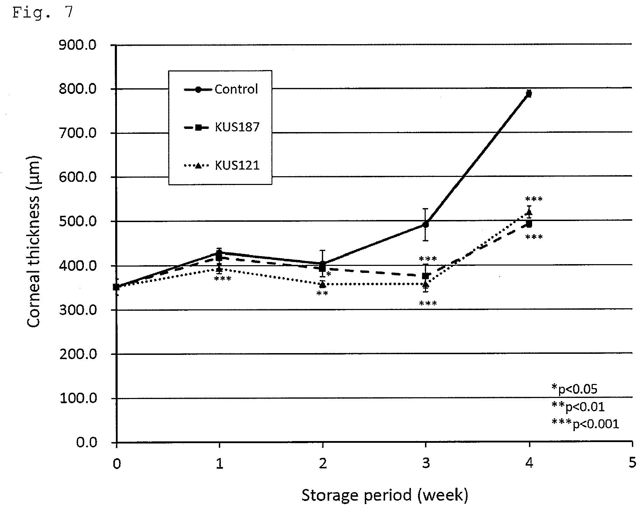

The tissue sections were stained with HE-staining and observed by microscopy. The images were compared over time among the preservation conditions (Fig. 6). Microscopic images of the sections including the central parts of the corneas were obtained and the central corneal thicknesses were measured and compared over time among the preservation conditions (Fig. 7). In the corneas preserved in Optisol-GS (control), the corneal endothelial cells swelled at week 1 and the corneal stroma swelled at week 3. In the presence of KUS121 or KUS187, the cell swelling was suppressed until week 2 and the corneal stroma swelling was kept mild until week 4. The corneal epitheliums and the cornea thicknesses were maintained better in the KUS121 and KUS187 groups than in the control group, especially at week 3 or later. The results suggest that KUS121 and KUS187 protect corneal epithelial cells and corneal endothelial cells.

### (2) Apoptosis of corneal epithelial cells assessed by TUNEL assay

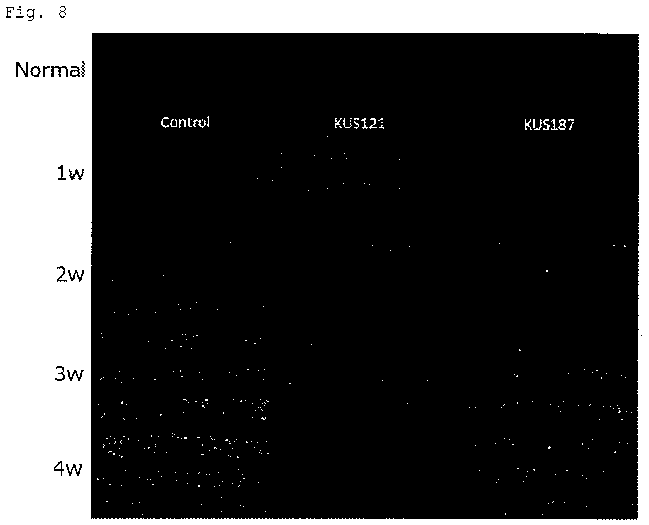

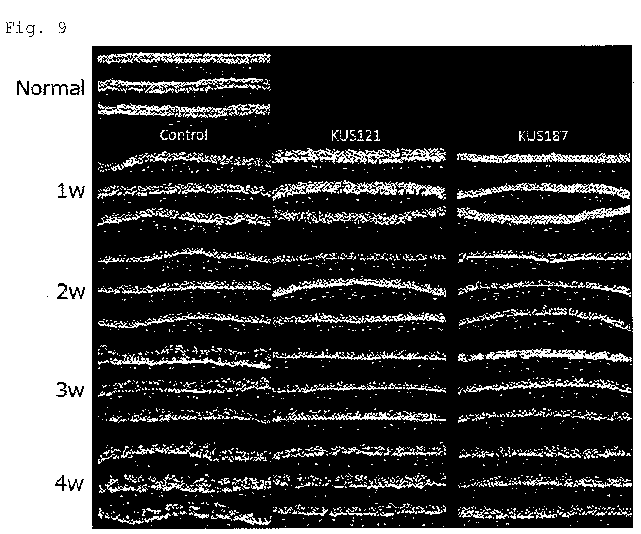

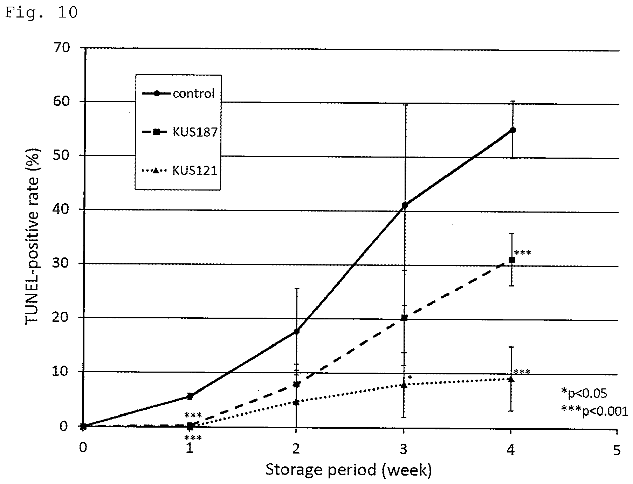

The tissue sections were fluorescently stained by TUNEL method and observed by confocal microscopy. Images of the epithelial layers around the centers of the corneas were obtained in three different fields (Fig. 8). Fig. 9 shows the iodopropidium (PI)-stained images of the same fields. The total corneal epithelial cells stained with PI and the TUNEL-positive cells were counted and the percentages of the TUNEL-positive cells were calculated. The percentages were compared over time among the preservation conditions (Fig. 10). The TUNEL-positive apoptotic cells were increased in the corneal epithelial cells of the control group after week 1. The apoptosis was suppressed in the KUS121 and KUS187 groups. The TUNEL-positive cells in the KUS121 and KUS187 groups were fewer through the test period than in the control group. The results suggest that KUS121 and KUS187 suppress apoptosis of corneal epithelial cells.

### (3) Function of corneal endothelia assessed by immunofluorescence staining of Na+/K+ ATPase

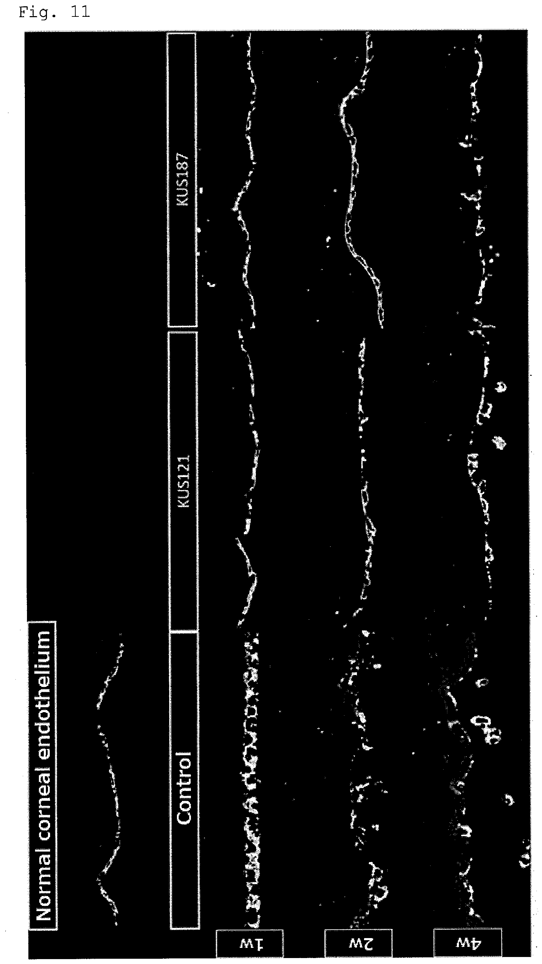

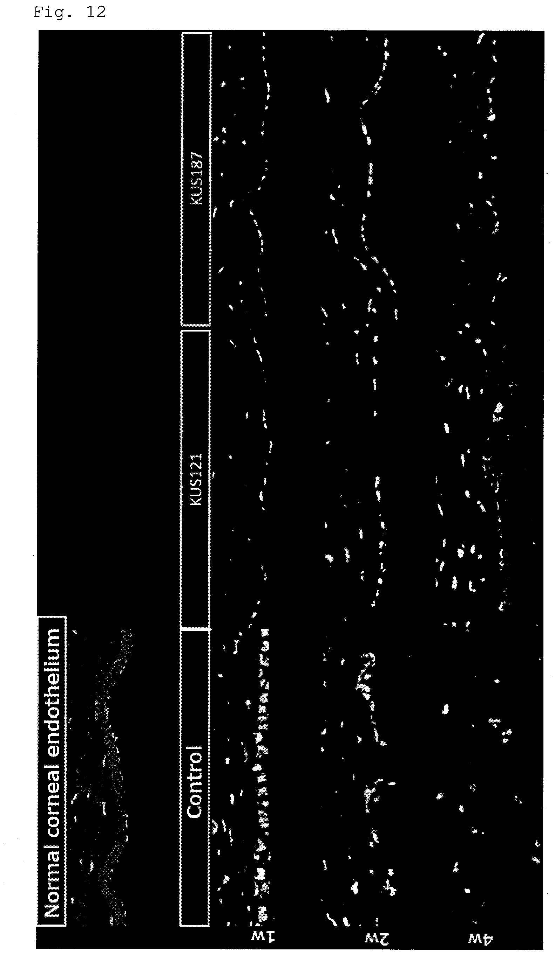

The tissue sections were fluorescently stained with anti-Na+/K+ ATPase antibody, the corneal endothelia around the centers of the corneas were observed by indirect immunofluorescence microscopy, and the images were compared over time among the preservation conditions (Fig. 11). Fig. 12 shows the PI-stained images of the same fields. The corneal endothelial cells were maintained better in the KUS121 and KUS187 groups than in the control group from week 1. The results suggest that KUS121 and KUS187 protect corneal endothelial cells from the earlier stage.

### (4) Electron microscopy

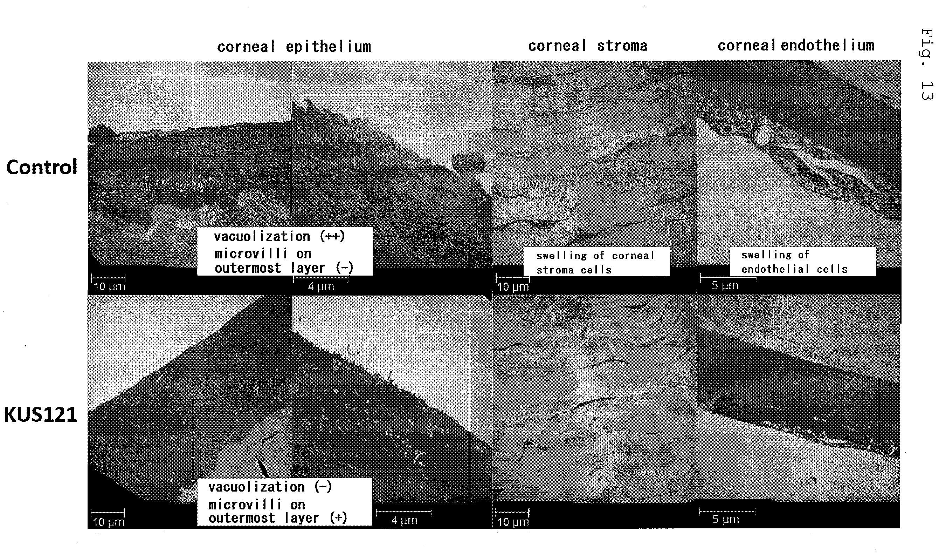

Corneas preserved for one week were immersed in 0.1 M phosphate buffer containing 2% glutaraldehyde at 4°C for prefixation, in 0.1 M phosphate buffer at 4°C overnight for washing, and in 2% aqueous osmium solution at 4°C for 3 hours for postfixation. For dehydration the corneas were successively immersed in ethanol solutions of high concentrations (50, 70, 90, 100, 100, and 100%) at 4°C (50%) or a room temperature for 15 minutes. For substitution the corneas were immersed in propylene oxide at a room temperature for 45 minutes and then in a mixture of propylene oxide and epoxy resin at a room temperature for 1.5 hours. For embedding the samples and epoxy resin were put into capsules and cured at 60°C for 48 hours. Then 80-90 nm sections were prepared by using an ultramicrotome and put on 200 meshes. The sections were stained with 2% uranyl acetate and lead and reinforced by carbon deposition (reinforcement of sections against electron beams). The samples were observed by transmission electron microscopy and images were obtained (Fig. 13).

Vacuolization of the corneal epithelial cells and loss of microvilli were observed in the control, but not in the corneas preserved in a solution containing KUS121. Swelling of the corneal stroma and the corneal endothelial cells was observed in the control, but not in the corneas preserved in a solution containing KUS121. The results suggest that KUS121 protects corneal epithelial cells, corneal stroma, and corneal endothelial cells from the earlier stage.

The results of Tests 1 to 3 revealed that KUS121 and KUS187 protect corneal endothelial cells and corneal epithelial cells and suppress cell death, and that the pump function is normal and corneal edema is suppressed in the corneal endothelial cells protected from the cell death.

### INDUSTRIAL APPLICABILITY

The composition disclosed herein may be widely used in the field of ophthalmic medicine.

## Claims

1. A composition for use for the protection of a cornea, comprising a compound of formula (I): 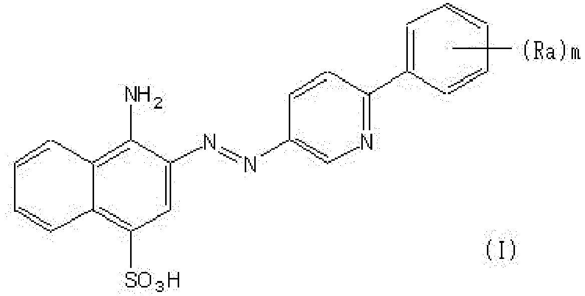 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt or solvate thereof.

2. The composition for use according to claim 1, wherein each Ra radical is independently selected from the group consisting of halo, hydroxy, alkyl, halo-substituted alkyl, and alkoxy.

3. The composition for use according to claim 1, wherein the compound of formula (I) is selected from the group consisting of
4-amino-3-(6-phenylpyridine-3-ylazo)naphthalene-1-sulfonic acid;
4-amino-3-(6-p-tolylpyridine-3-ylazo)naphthalene-1-sulfonic acid;
4-amino-3-(6-m-tolylpyridine-3-ylazo)naphthalene-1-sulfonic acid;
4-amino-3-(6-o-tolylpyridine-3-ylazo)naphthalene-1-sulfonic acid;
4-amino-3-(6-biphenyl-2-ylpyridine-3-ylazo)naphthalene-1-sulfonic acid;
3-[6-(2-acetylphenyl)pyridine-3-ylazo]-4-aminonaphthalene-1-sulfonic acid;
3-[6-(3-acetylphenyl)pyridine-3-ylazo]-4-aminonaphthalene-1-sulfonic acid;
3-[6-(4-acetylphenyl)pyridine-3-ylazo]-4-aminonaphthalenesulfonic acid;
4-amino-3-[6-(2,4-dichlorophenyl)pyridine-3-ylazo]naphthalene-1-sulfonic acid;
4-amino-3-[6-(2-trifluoromethylphenyl)pyridine-3-ylazo]naphthalene-1-sulfonic acid;
4-amino-3-[6-(4-trifluoromethylphenyl)pyridine-3-ylazo]naphthalene-1-sulfonic acid;
4-amino-3-[6-(2-chlorophenyl)pyridine-3-ylazo]naphthalene-1-sulfonic acid;
4-amino-3-[6-(3-chlorophenyl)pyridine-3-ylazo]naphthalene-1-sulfonic acid;
4-amino-3-[6-(4-chlorophenyl)pyridine-3-ylazo]naphthalene-1-sulfonic acid;
4-amino-3-[6-(2-methoxyphenyl)pyridine-3-ylazo]naphthalene-1-sulfonic acid;
4-amino-3-[6-(4-methoxyphenyl)pyridine-3-ylazo]naphthalene-1-sulfonic acid;
4-amino-3-[6-(2-isopropoxyphenyl)pyridine-3-ylazo]naphthalene-1-sulfonic acid;
4-amino-3-[6-(4-isopropoxyphenyl)pyridine-3-ylazo]naphthalene-1-sulfonic acid;
4-amino-3-[6-(2-phenoxyphenyl)pyridine-3-ylazo]naphthalene-1-sulfonic acid;
4-amino-3-[6-(3-methoxyphenyl)pyridine-3-ylazo]naphthalene-1-sulfonic acid;
4-amino-3-[6-(2,3-dimethylphenyl)pyridine-3-ylazo]naphthalene-1-sulfonic acid;
4-amino-3-[6-(2,5-dimethylphenyl)pyridine-3-ylazo]naphthalene-1-sulfonic acid;
4-amino-3-[6-(3,5-dimethylphenyl)pyridine-3-ylazo]naphthalene-1-sulfonic acid;
4-amino-3-[6-(3-trifluoromethylphenyl)pyridine-3-ylazo]naphthalene-1-sulfonic acid;
4-{4-[5-(1-amino-4-sulfonaphthalene-2-ylazo)pyridine-2-yl]phenyl}-4-oxobutyric acid;
4-amino-3-(6-biphenyl-3-ylpyridine-3-ylazo)naphthalene-1-sulfonic acid;
4-amino-3-[6-(3-cyanophenyl)pyridine-3-ylazo]naphthalene-1-sulfonic acid;
4-amino-3-[6-(4-cyanophenyl)pyridine-3-ylazo]naphthalene-1-sulfonic acid;
4-amino-3-[6-(3,5-bistrifluoromethylphenyl)pyridine-3-ylazo]naphthalenesulfonic acid;
4-amino-3-[6-(4-benzoylphenyl)pyridine-3-ylazo]naphthalene-1-sulfonic acid;
4-amino-3-[6-(2-propoxyphenyl)pyridine-3-ylazo]naphthalene-1-sulfonic acid;
4-amino-3-[6-(4-fluoro-2-methylphenyl)pyridine-3-ylazo]naphthalene-1-sulfonic acid;
4-amino-3-[6-(5-fluoro-2-propoxyphenyl)pyridine-3-ylazo]naphthalene-1-sulfonic acid;
4-amino-3-[6-(2-fluoro-6-propoxyphenyl)pyridine-3-ylazo]naphthalene-1-sulfonic acid;
4-amino-3-[6-(4-fluoro-2-propoxyphenyl)pyridine-3-ylazo]naphthalene-1-sulfonic acid;
4-amino-3-[6-(5-fluoro-2-methylphenyl)pyridine-3-ylazo]naphthalene-1-sulfonic acid;
4-amino-3-[6-(2-fluoro-5-methylphenyl)pyridine-3-ylazo]naphthalene-1-sulfonic acid;
4-amino-3-[6-(2-butoxyphenyl)pyridine-3-ylazo]naphthalene-1-sulfonic acid;
4-amino-3-[6-(2-hexyloxyphenyl)pyridine-3-ylazo]naphthalene-1-sulfonic acid;
4-amino-3-[6-(4-butylphenyl)pyridine-3-ylazo]naphthalene-1-sulfonic acid;
4-amino-3-[6-(2-hydroxyphenyl)pyridine-3-ylazo]naphthalene-1-sulfonic acid;
4-amino-3-{6-[2-(6-hydroxyhexyloxy)phenyl]pyridine-3-ylazo}naphthalene-1-sulfonic acid;
4-{2-[5-(1-amino-4-sulfonaphthalene-2-ylazo)pyridine-2-yl]phenoxy} butyric acid;
4-amino-3-{6-[2-(3-hydroxypropoxy)phenyl]pyridine-3-ylazo}naphthalene-1-sulfonic acid;
4-amino-3-[6-(2-isobutoxyphenyl)pyridine-3-ylazo]naphthalene-1-sulfonic acid;
4-amino-3-[6-(5-chloro-2-hydroxyphenyl)pyridine-3-ylazo]naphthalene-1-sulfonic acid;
4-amino-3-[6-(4-methylbiphenyl-2-yl)pyridine-3-ylazo]naphthalene-1-sulfonic acid;
4-amino-3-[6-(4'-chloro-4-methylbiphenyl-2-yl)pyridine-3-ylazo]naphthalene-1-sulfonic acid;
4-amino-3-[6-(4,3',5'-trimethylbiphenyl-2-yl)pyridine-3-ylazo]naphthalene-1-sulfonic acid;
4-amino-3-[6-(3'-chloro-4-methylbiphenyl-2-yl)pyridine-3-ylazo]naphthalene-1-sulfonic acid;
4-amino-3-[6-(2,6-dimethylphenyl)pyridine-3-ylazo]naphthalene-1-sulfonic acid;
4-amino-3-[6-(3-formyl-2-isopropoxy-5-methylphenyl)pyridine-3-ylazo]naphthalene-1-sulfonic acid; and
4-amino-3-[6-(3-formyl-2-butoxy-5-methylphenyl)pyridine-3-ylazo]naphthalene-1-sulfonic acid.

4. The composition for use according to claim 1, wherein the compound of formula (I) is 4-amino-3-[6-(4-fluoro-2-methylphenyl)pyridine-3-ylazo]naphthalene-1-sulfonic acid or 4-amino-3-[6-(3-formyl-2-butoxy-5-methylphenyl)pyridine-3-ylazo]naphthalene-1-sulfonic acid.

5. The composition for use according to any one of claims 1 to 4, wherein the composition is for treating or preventing a corneal disease.

6. The composition for use according to claim 5, wherein the corneal disease is a corneal endothelial disease.

7. The composition for use according to claim 5, wherein the corneal disease is a corneal epithelial disease.

8. The composition for use according to claim 5, wherein the corneal disease is cornea guttata, Fuchs endothelial corneal dystrophy, posterior polymorphous corneal dystrophy, iridocorneal endothelial syndrome, congenital hereditary corneal endothelial dystrophy, cytomegalovirus endotheliitis, herpetic endotheliitis, exfoliation syndrome, corneal endothelial graft rejection, keratouveitis, interstitial keratitis, corneal endotheliitis, corneal endothelial cell loss after corneal transplantation, corneal injury after intraocular surgery, corneal injury induced by glaucomatous attack, corneal injury caused by long-term contact lens use, corneal trauma, intrapartum corneal trauma, bullous keratopathy, superficial punctate keratitis, corneal erosion, corneal ulcer, dry eye, keratoconjunctivitis sicca, or corneal marginal ulcer.

9. The composition for use according to claim 5, wherein the corneal disease is Fuchs endothelial corneal dystrophy, bullous keratopathy, corneal erosion, dry eye, or corneal trauma.

10. The composition for use according to any one of claims 1 to 9, wherein the composition is an eye drop.

11. The composition for use according to any one of claims 1 to 9, wherein the composition is an ocular irrigation solution or a composition to be added to an ocular irrigation solution.

12. A pharmaceutical composition for use for treating or preventing a corneal disease, comprising a compound of formula (I): 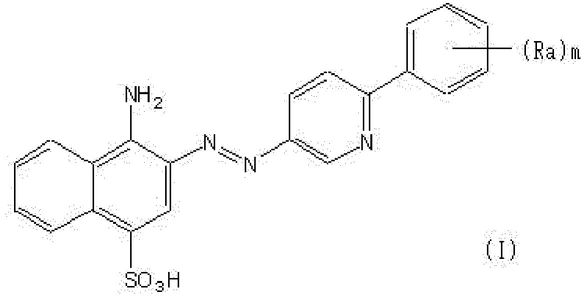 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt or solvate thereof.

13. A composition for use for ocular irrigation, comprising a compound of formula (I): 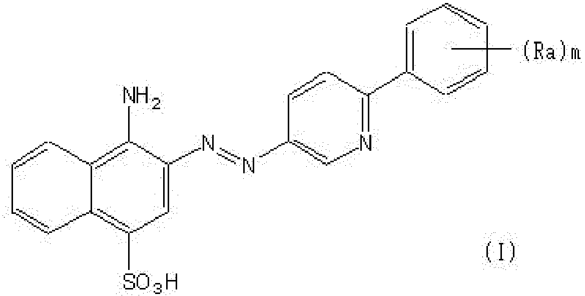 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt or solvate thereof.

14. Use of a composition for preserving a corneal graft, the composition comprising a compound of formula (I): 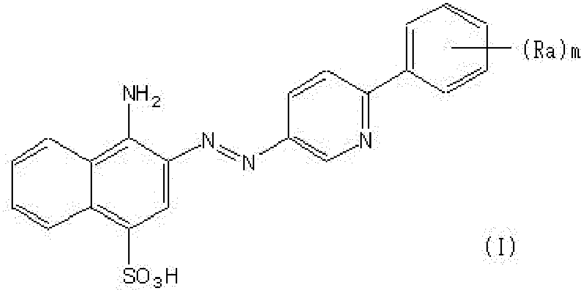 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt or solvate thereof.

15. A method for preserving or protecting a corneal graft, comprising preserving a corneal graft in a corneal preservation solution comprising a compound of formula (I): 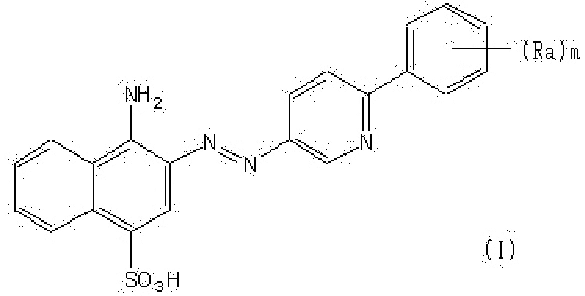 wherein
Ra is selected from the group consisting of halo, hydroxy, alkyl, halo-substituted alkyl, aryl, halo- or alkyl-substituted aryl, alkoxy, hydroxy- or carboxy-substituted alkoxy, aryloxy, halo- or alkyl-substituted aryloxy, CHO, C(O)-alkyl, C(O)-aryl, C(O)-alkyl-carboxyl, C(O)-alkylene-carboxy ester, and cyano, and
m is an integer selected from 0 to 4,
or an ester, oxide, pharmaceutically acceptable salt or solvate thereof.

## Patentansprüche

1. Zusammensetzung zur Verwendung zum Schutz einer Hornhaut, umfassend eine Verbindung der Formel (I): 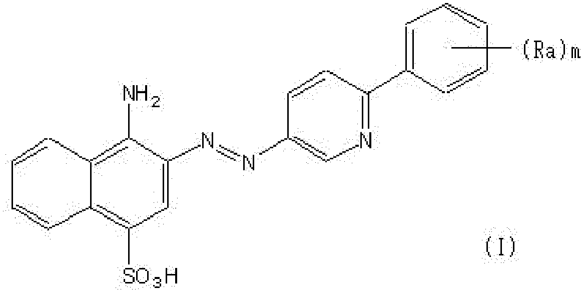 worin
Ra aus der Gruppe, bestehend aus Halogen, Hydroxy, Alkyl, Halogen-substituiertem Alkyl, Aryl, Halogen- oder Alkyl-substituiertem Aryl, Alkoxy, Hydroxy- oder Carboxy-substituiertem Alkoxy, Aryloxy, Halogen- oder Alkyl-substituiertem Aryloxy, CHO, C(O)-Alkyl, C(O)-Aryl, C(O)-Alkylcarboxyl, C(O)-Alkylencarboxyester und Cyano, ausgewählt ist und
m eine ganze Zahl, ausgewählt aus 0 bis 4, ist,
oder einen Ester, ein Oxid, ein pharmazeutisch annehmbares Salz oder ein Solvat davon.

2. Zusammensetzung zur Verwendung gemäß Anspruch 1, wobei jeder Ra-Rest unabhängig aus der Gruppe, bestehend aus Halogen, Hydroxy, Alkyl, Halogen-substituiertem Alkyl und Alkoxy, ausgewählt ist.

3. Zusammensetzung zur Verwendung gemäß Anspruch 1, wobei die Verbindung der Formel (I) aus der Gruppe, bestehend aus
4-Amino-3-(6-phenylpyridin-3-ylazo)naphthalin-1-sulfonsäure,
4-Amino-3-(6-p-tolylpyridin-3-ylazo)naphthalin-1-sulfonsäure,
4-Amino-3-(6-m-tolylpyridin-3-ylazo)naphthalin-1-sulfonsäure,
4-Amino-3-(6-o-tolylpyridin-3-ylazo)naphthalin-1-sulfonsäure,
4-Amino-3-(6-biphenyl-2-ylpyridin-3-ylazo)naphthalin-1-sulfonsäure,
3-[6-(2-Acetylphenyl)pyridin-3-ylazo]-4-aminonaphthalin-1-sulfonsäure,
3-[6-(3-Acetylphenyl)pyridin-3-ylazo]-4-aminonaphthalin-1-sulfonsäure,
3-[6-(4-Acetylphenyl)pyridin-3-ylazo]-4-aminonaphthalinsulfonsäure,
4-Amino-3-[6-(2,4-dichlorphenyl)pyridin-3-ylazo]naphthalin-1-sulfonsäure,
4-Amino-3-[6-(2-trifluormethylphenyl)pyridin-3-ylazo]naphthalin-1-sulfonsäure,
4-Amino-3-[6-(4-trifluormethylphenyl)pyridin-3-ylazo]naphthalin-1-sulfonsäure,
4-Amino-3-[6-(2-chlorphenyl)pyridin-3-ylazo]naphthalin-1-sulfonsäure,
4-Amino-3-[6-(3-chlorphenyl)pyridin-3-ylazo]naphthalin-1-sulfonsäure,
4-Amino-3-[6-(4-chlorphenyl)pyridin-3-ylazo]naphthalin-1-sulfonsäure,
4-Amino-3-[6-(2-methoxyphenyl)pyridin-3-ylazo]naphthalin-1-sulfonsäure,
4-Amino-3-[6-(4-methoxyphenyl)pyridin-3-ylazo]naphthalin-1-sulfonsäure,
4-Amino-3-[6-(2-isopropoxyphenyl)pyridin-3-ylazo]naphthalin-1-sulfonsäure,
4-Amino-3-[6-(4-isopropoxyphenyl)pyridin-3-ylazo]naphthalin-1-sulfonsäure,
4-Amino-3-[6-(2-phenoxyphenyl)pyridin-3-ylazo]naphthalin-1-sulfonsäure,
4-Amino-3-[6-(3-methoxyphenyl)pyridin-3-ylazo]naphthalin-1-sulfonsäure,
4-Amino-3-[6-(2,3-dimethylphenyl)pyridin-3-ylazo]naphthalin-1-sulfonsäure,
4-Amino-3-[6-(2,5-dimethylphenyl)pyridin-3-ylazo]naphthalin-1-sulfonsäure,
4-Amino-3-[6-(3,5-dimethylphenyl)pyridin-3-ylazo]naphthalin-1-sulfonsäure,
4-Amino-3-[6-(3-trifluormethylphenyl)pyridin-3-ylazo]naphthalin-1-sulfonsäure,
4-{4-[5-(1-Amino-d-sulfonaphthalin-2-ylazo)pyridin-2-yl]phenyl}-4-oxobuttersäure,
4-Amino-3-(6-biphenyl-3-ylpyridin-3-ylazo)naphthalin-1-sulfonsäure,
4-Amino-3-[6-(3-cyanophenyl)pyridin-3-ylazo]naphthalin-1-sulfonsäure,
4-Amino-3-[6-(4-cyanophenyl)pyridin-3-ylazo]naphthalin-1-sulfonsäure,
4-Amino-3-[6-(3,5-bistrifluormethylphenyl)pyridin-3-ylazo]naphthalinsulfonsäure,
4-Amino-3-[6-(4-benzoylphenyl)pyridin-3-ylazo]naphthalin-1-sulfonsäure,
4-Amino-3-[6-(2-propoxyphenyl)pyridin-3-ylazo]naphthalin-1-sulfonsäure,
4-Amino-3-[6-(4-fluor-2-methylphenyl)pyridin-3-ylazo]naphthalin-1-sulfonsäure,
4-Amino-3-[6-(5-fluor-2-propoxyphenyl)pyridin-3-ylazo]naphthalin-1-sulfonsäure,
4-Amino-3-[6-(2-fluor-6-propoxyphenyl)pyridin-3-ylazo]naphthalin-1-sulfonsäure,
4-Amino-3-[6-(4-fluor-2-propoxyphenyl)pyridin-3-ylazo]naphthalin-1-sulfonsäure,
4-Amino-3-[6-(5-fluor-2-methylphenyl)pyridin-3-ylazo]naphthalin-1-sulfonsäure,
4-Amino-3-[6-(2-fluor-5-methylphenyl)pyridin-3-ylazo]naphthalin-1-sulfonsäure,
4-Amino-3-[6-(2-butoxyphenyl)pyridin-3-ylazo]naphthalin-1-sulfonsäure,
4-Amino-3-[6-(2-hexyloxyphenyl)pyridin-3-ylazo]naphthalin-1-sulfonsäure,
4-Amino-3-[6-(4-butylphenyl)pyridin-3-ylazo]naphthalin-1-sulfonsäure,
4-Amino-3-[6-(2-hydroxyphenyl)pyridin-3-ylazo]naphthalin-1-sulfonsäure,
4-Amino-3-{6-[2-(6-hydroxyhexyloxy)phenyl]pyridin-3-ylazo}naphthalin-1-sulfonsäure,
4-{2-[5-(1-Amino-4-sulfonaphthalin-2-ylazo)pyridin-2-yl]phenoxy}buttersäure,
4-Amino-3-{6-[2-(3-hydroxypropoxy)phenyl]pyridin-3-ylazo}naphthalin-1-sulfonsäure,
4-Amino-3-[6-(2-isobutoxyphenyl)pyridin-3-ylazo]naphthalin-1-sulfonsäure,
4-Amino-3-[6-(5-chlor-2-hydroxyphenyl)pyridin-3-ylazo]naphthalin-1-sulfonsäure,
4-Amino-3-[6-(4-methylbiphenyl-2-yl)pyridin-3-ylazo]naphthalin-1-sulfonsäure,
4-Amino-3-[6-(4'-chlor-4-methylbiphenyl-2-yl)pyridin-3-ylazo]naphthalin-1-sulfonsäure,
4-Amino-3-[6-(4,3',5'-trimethylbiphenyl-2-yl)pyridin-3-ylazo]naphthalin-1-sulfonsäure,
4-Amino-3-[6-(3'-chlor-4-methylbiphenyl-2-yl)pyridin-3-ylazo]naphthalin-1-sulfonsäure,
4-Amino-3-[6-(2,6-dimethylphenyl)pyridin-3-ylazo]naphthalin-1-sulfonsäure,
4-Amino-3-[6-(3-formyl-2-isopropoxy-5-methylphenyl)pyridin-3-ylazo]naphthalin-1-sulfonsäure und
4-Amino-3-[6-(3-formyl-2-butoxy-5-methylphenyl)pyridin-3-ylazo]naphthalin-1-sulfonsäure, ausgewählt ist.

4. Zusammensetzung zur Verwendung gemäß Anspruch 1, wobei die Verbindung der Formel (I) 4-Amino-3-[6-(4-fluor-2-methylphenyl)pyridin-3-ylazo]naphthalin-1-sulfonsäure oder 4-Amino-3-[6-(3-formyl-2-butoxy-5-methylphenyl)pyridin-3-ylazo]naphthalin-1-sulfonsäure ist.

5. Zusammensetzung zur Verwendung gemäß irgendeinem der Ansprüche 1 bis 4, wobei die Zusammensetzung zur Behandlung oder Vorbeugung einer Hornhauterkrankung dient.

6. Zusammensetzung zur Verwendung gemäß Anspruch 5, wobei die Hornhauterkrankung eine Hornhautendothelerkrankung ist.

7. Zusammensetzung zur Verwendung gemäß Anspruch 5, wobei die Hornhauterkrankung eine Hornhautepithelerkrankung ist.

8. Zusammensetzung zur Verwendung gemäß Anspruch 5, wobei die Hornhauterkrankung Cornea guttata, Fuchs-Hornhautendotheldystrophie, Hintere polymorphe Hornhautdystrophie, iridokorneales endotheliales Syndrom, kongenitale hereditäre Hornhautendotheldystrophie, Cytomegalovirus-Endotheliitis, herpetische Endotheliitis, Exfoliationssyndrom, Hornhautendothe1-Transplantatabstoßung, Keratouveitis, interstitielle Keratitis, Hornhautendotheliitis, Hornhautendothelzellenverlust nach Hornhauttransplantation, Hornhautverletzung nach intraokularer Chirurgie, durch einen Glaukomanfall induzierte Hornhautverletzung, durch langfristige Kontaktlinsenverwendung verursachte Hornhautverletzung, Trauma der Hornhaut, intrapartales Trauma der Hornhaut, bullöse Keratopathie, Keratitis superficialis punctata, Hornhauterosion, Hornhautgeschwür, Trockenes Auge, Keratokonjunktivitis sicca oder periphäre Hornhautulzeration ist.

9. Zusammensetzung zur Verwendung gemäß Anspruch 5, wobei die Hornhauterkrankung Fuchs-Hornhautendotheldystrophie, bullöse Keratopathie, Hornhauterosion, Trockenes Auge oder Trauma der Hornhaut ist.

10. Zusammensetzung zur Verwendung gemäß einem der Ansprüche 1 bis 9, wobei die Zusammensetzung ein Augentropfen ist.

11. Zusammensetzung zur Verwendung gemäß einem der Ansprüche 1 bis 9, wobei die Zusammensetzung eine Augenspüllösung oder eine Zusammensetzung, die einer Augenspüllösung hinzugefügt wird, ist.

12. Pharmazeutische Zusammensetzung zur Verwendung bei der Behandlung oder Vorbeugung einer Hornhauterkrankung, umfassend eine Verbindung der Formel (I): 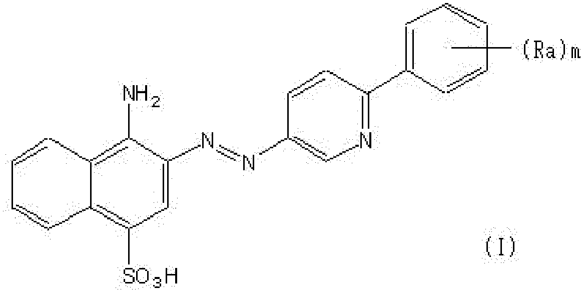 worin
Ra aus der Gruppe, bestehend aus Halogen, Hydroxy, Alkyl, Halogen-substituiertem Alkyl, Aryl, Halogen- oder Alkyl-substituiertem Aryl, Alkoxy, Hydroxy- oder Carboxy-substituiertem Alkoxy, Aryloxy, Halogen- oder Alkyl-substituiertem Aryloxy, CHO, C(O)-Alkyl, C(O)-Aryl, C(O)-Alkylcarboxyl, C(O)-Alkylencarboxyester und Cyano, ausgewählt ist und
m eine ganze Zahl, ausgewählt aus 0 bis 4, ist,
oder einen Ester, ein Oxid, ein pharmazeutisch annehmbares Salz oder ein Solvat davon.

13. Zusammensetzung zur Verwendung zur Augenspülung, umfassend eine Verbindung der Formel (I): 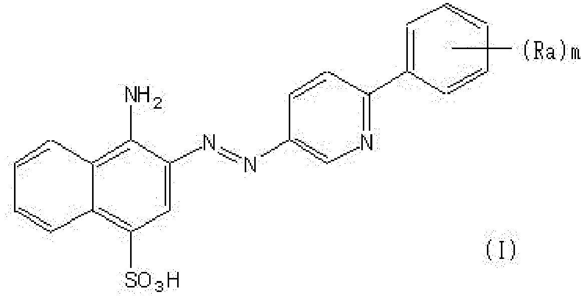 worin
Ra aus der Gruppe, bestehend aus Halogen, Hydroxy, Alkyl, Halogen-substituiertem Alkyl, Aryl, Halogen- oder Alkyl-substituiertem Aryl, Alkoxy, Hydroxy- oder Carboxy-substituiertem Alkoxy, Aryloxy, Halogen- oder Alkyl-substituiertem Aryloxy, CHO, C(O)-Alkyl, C(O)-Aryl, C(O)-Alkylcarboxyl, C(O)-Alkylencarboxyester und Cyano, ausgewählt ist und
m eine ganze Zahl, ausgewählt aus 0 bis 4, ist,
oder einen Ester, ein Oxid, ein pharmazeutisch annehmbares Salz oder ein Solvat davon.

14. Verwendung einer Zusammensetzung zum Konservieren eines Hornhauttransplantats, wobei die Zusammensetzung eine Verbindung der Formel (I): 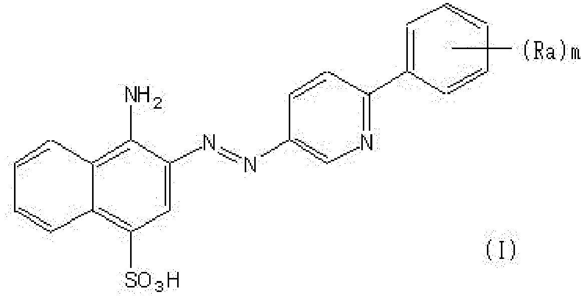 worin
Ra aus der Gruppe, bestehend aus Halogen, Hydroxy, Alkyl, Halogen-substituiertem Alkyl, Aryl, Halogen- oder Alkyl-substituiertem Aryl, Alkoxy, Hydroxy- oder Carboxy-substituiertem Alkoxy, Aryloxy, Halogen- oder Alkyl-substituiertem Aryloxy, CHO, C(O)-Alkyl, C(O)-Aryl, C(O)-Alkylcarboxyl, C(O)-Alkylencarboxyester und Cyano, ausgewählt ist und
m eine ganze Zahl, ausgewählt aus 0 bis 4, ist,
oder einen Ester, ein Oxid, ein pharmazeutisch annehmbares Salz oder ein Solvat davon umfasst.

15. Verfahren zum Konservieren oder Schützen eines Hornhauttransplantats, umfassend das Konservieren eines Hornhauttransplantats in einer Hornhautkonservierungslösung, umfassend eine Verbindung der Formel (I): 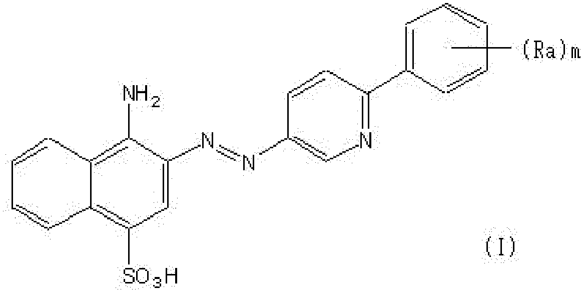 worin
Ra aus der Gruppe, bestehend aus Halogen, Hydroxy, Alkyl, Halogen-substituiertem Alkyl, Aryl, Halogen- oder Alkyl-substituiertem Aryl, Alkoxy, Hydroxy- oder Carboxy-substituiertem Alkoxy, Aryloxy, Halogen- oder Alkyl-substituiertem Aryloxy, CHO, C(O)-Alkyl, C(O)-Aryl, C(O)-Alkylcarboxyl, C(O)-Alkylencarboxyester und Cyano, ausgewählt ist und
m eine ganze Zahl, ausgewählt aus 0 bis 4, ist,
oder einen Ester, ein Oxid, ein pharmazeutisch annehmbares Salz oder ein Solvat davon.

## Revendications

1. Composition à utiliser pour la protection d'une cornée, comprenant un composé de formule (I) : 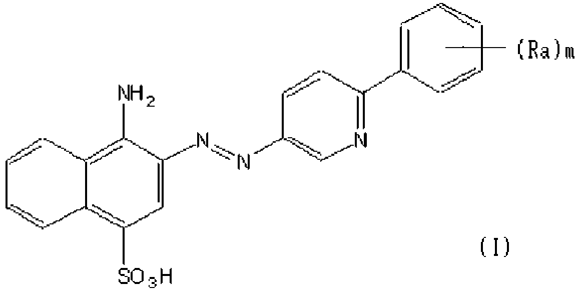 dans laquelle
Ra est choisi dans le groupe consistant en un halo, un hydroxy, un alkyle, un alkyle substitué par un halo, un aryle, un aryle substitué par un halo ou un alkyle, un alcoxy, un alcoxy substitué par un hydroxy ou un carboxy, un aryloxy, un aryloxy substitué par un halo ou un alkyle, CHO, C(O)-alkyle, C(O)-aryle, C(O)-alkyl-carboxyle, ester de C(O)-alkylène-carboxy, et cyano, et
m est un entier choisi de 0 à 4,
ou un ester, oxyde, sel ou solvate pharmaceutiquement acceptable de celui-ci.

2. Composition à utiliser selon la revendication 1, dans laquelle chaque radical Ra est choisi indépendamment dans le groupe consistant en un halo, un hydroxy, un alkyle, un alkyle substitué par un halo, et un alcoxy.

3. Composition à utiliser selon la revendication 1, dans laquelle le composé de formule (I) est choisi dans le groupe consistant en
l'acide 4-amino-3-(6-phénylpyridine-3-ylazo)naphtalène-1-sulfonique ;
l'acide 4-amino-3-(6-p-tolylpyridine-3-ylazo)naphtalène-1-sulfonique ;
l'acide 4-amino-3-(6-m-tolylpyridine-3-ylazo)naphtalène-1-sulfonique ;
l'acide 4-amino-3-(6-o-tolylpyridine-3-ylazo)naphtalène-1-sulfonique ;
l'acide 4-amino-3-(6-biphényl-2-ylpyridine-3-ylazo)naphtalène-1-sulfonique ;
l'acide 3-[6-(2-acétylphényl)pyridine-3-ylazo]-4-aminonaphtalène-1-sulfonique ;
l'acide 3-[6-(3-acétylphényl)pyridine-3-ylazo]-4-aminonaphtalène-1-sulfonique ;
l'acide 3-[6-(4-acétylphényl)pyridine-3-ylazo]-4-aminonaphtalènesulfonique ;
l'acide 4-amino-3-[6-(2,4-dichlorophényl)pyridine-3-ylazo]naphtalène-1-sulfonique ;
l'acide 4-amino-3-[6-(2-trifluorométhylphényl)pyridine-3-ylazo]naphtalène-1-sulfonique ;
l'acide 4-amino-3-[6-(4-trifluorométhylphényl)pyridine-3-ylazo]naphtalène-1-sulfonique ;
l'acide 4-amino-3-[6-(2-chlorophényl)pyridine-3-ylazo]naphtalène-1-sulfonique ;
l'acide 4-amino-3-[6-(3-chlorophényl)pyridine-3-ylazo]naphtalène-1-sulfonique ;
l'acide 4-amino-3-[6-(4-chlorophényl)pyridine-3-ylazo]naphtalène-1-sulfonique ;
l'acide 4-amino-3-[6-(2-méthoxyphényl)pyridine-3-ylazo]naphtalène-1-sulfonique ;
l'acide 4-amino-3-[6-(4-méthoxyphényl)pyridine-3-ylazo]naphtalène-1-sulfonique ;
l'acide 4-amino-3-[6-(2-isopropoxyphényl)pyridine-3-ylazo]naphtalène-1-sulfonique ;
l'acide 4-amino-3-[6-(4-isopropoxyphényl)pyridine-3-ylazo]naphtalène-1-sulfonique ;
l'acide 4-amino-3-[6-(2-phénoxyphényl)pyridine-3-ylazo]naphtalène-1-sulfonique ;
l'acide 4-amino-3-[6-(3-méthoxyphényl)pyridine-3-ylazo]naphtalène-1-sulfonique ;
l'acide 4-amino-3-[6-(2,3-diméthylphényl)pyridine-3-ylazo]naphtalène-1-sulfonique ;
l'acide 4-amino-3-[6-(2,5-diméthylphényl)pyridine-3-ylazo]naphtalène-1-sulfonique ;
l'acide 4-amino-3-[6-(3,5-diméthylphényl)pyridine-3-ylazo]naphtalène-1-sulfonique ;
l'acide 4-amino-3-[6-(3-trifluorométhylphényl)pyridine-3-ylazo]naphtalène-1-sulfonique ;
l'acide 4-{4-[5-(1-amino-4-sulfonaphtalène-2-ylazo)pyridine-2-yl]phényl}-4-oxobutyrique ;
l'acide 4-amino-3-(6-biphényl-3-ylpyridine-3-ylazo)naphtalène-1-sulfonique ;
l'acide 4-amino-3-[6-(3-cyanophényl)pyridine-3-ylazo]naphtalène-1-sulfonique ;
l'acide 4-amino-3-[6-(4-cyanophényl)pyridine-3-ylazo]naphtalène-1-sulfonique ;
l'acide 4-amino-3-[6-(3,5-bistrifluorométhylphényl)pyridine-3-ylazo]naphtalène 1 sulfonique ;
l'acide 4-amino-3-[6-(4-benzoylphényl)pyridine-3-ylazo]naphtalène-1-sulfonique ;
l'acide 4-amino-3-[6-(2-propoxyphényl)pyridine-3-ylazo]naphtalène-1-sulfonique ;
l'acide 4-amino-3-[6-(4-fluoro-2-méthylphényl)pyridine-3-ylazo]naphtalène-1-sulfonique ;
l'acide 4-amino-3-[6-(5-fluoro-2-propoxyphényl)pyridine-3-ylazo]naphtalène-1-sulfonique ;
l'acide 4-amino-3-[6-(2-fluoro-6-propoxyphényl)pyridine-3-ylazo]naphtalène-1-sulfonique ;
l'acide 4-amino-3-[6-(4-fluoro-2-propoxyphényl)pyridine-3-ylazo]naphtalène-1-sulfonique ;
l'acide 4-amino-3-[6-(5-fluoro-2-méthylphényl)pyridine-3-ylazo]naphtalène-1-sulfonique ;
l'acide 4-amino-3-[6-(2-fluoro-5-méthylphényl)pyridine-3-ylazo]naphtalène-1-sulfonique ;
l'acide 4-amino-3-[6-(2-butoxyphényl)pyridine-3-ylazo]naphtalène-1-sulfonique ;
l'acide 4-amino-3-[6-(2-hexyloxyphényl)pyridine-3-ylazo]naphtalène-1-sulfonique ;
l'acide 4-amino-3-[6-(4-butylphényl)pyridine-3-ylazo]naphtalène-1-sulfonique ;
l'acide 4-amino-3-[6-(2-hydroxyphényl)pyridine-3-ylazo]naphtalène-1-sulfonique ;
l'acide 4-amino-3-{6-[2-(6-hydroxyhexyloxy)phényl]pyridine-3-ylazo}naphtalène-1-sulfonique ;
l'acide 4-{2-[5-(1-amino-4-sulfonaphtalène-2-ylazo)pyridine-2-yl]phénoxy}butyrique ;
l'acide 4-amino-3-{6-[2-(3-hydroxypropoxy)phényl]pyridine-3-ylazo}naphtalène-1-sulfonique ;
l'acide 4-amino-3-[6-(2-isobutoxyphényl)pyridine-3-ylazo]naphtalène-1-sulfonique ;
l'acide 4-amino-3-[6-(5-chloro-2-hydroxyphényl)pyridine-3-ylazo]naphtalène-1-sulfonique ;
l'acide 4-amino-3-[6-(4-méthylbiphényl-2-yl)pyridine-3-ylazo]naphtalène-1-sulfonique ;
l'acide 4-amino-3-[6-(4'-chloro-4-méthylbiphényl-2-yl)pyridine-3-ylazo]naphtalène-1-sulfonique ;
l'acide 4-amino-3-[6-(4,3',5'-triméthylbiphényl-2-yl)pyridine-3-ylazo]naphtalène-1-sulfonique ;
l'acide 4-amino-3-[6-(3'-chloro-4-méthylbiphényl-2-yl)pyridine-3-ylazo]naphtalène-1-sulfonique ;
l'acide 4-amino-3-[6-(2,6-diméthylphényl)pyridine-3-ylazo]naphtalène-1-sulfonique ;
l'acide 4-amino-3-[6-(3-formyl-2-isopropoxy-5-méthylphényl)pyridine-3-ylazo]naphtalène-1-sulfonique ; et
l'acide 4-amino-3-[6-(3-formyl-2-butoxy-5-méthylphényl)pyridine-3-ylazo]naphtalène-1-sulfonique.

4. Composition à utiliser selon la revendication 1, dans laquelle le composé de formule (I) est l'acide 4-amino-3-[6-(4-fluoro-2-méthylphényl)pyridine-3-ylazo]naphtalène-1-sulfonique ou l'acide 4-amino-3-[6-(3-formyl-2-butoxy-5-méthylphényl)pyridine-3-ylazo]naphtalène-1-sulfonique.

5. Composition à utiliser selon l'une quelconque des revendications 1 à 4, dans laquelle la composition est destinée au traitement ou à la prévention d'une maladie cornéenne.

6. Composition à utiliser selon la revendication 5, dans laquelle la maladie cornéenne est une maladie endothéliale cornéenne.

7. Composition à utiliser selon la revendication 5, dans laquelle la maladie cornéenne est une maladie épithéliale cornéenne.

8. Composition à utiliser selon la revendication 5, dans laquelle la maladie cornéenne est la cornée guttata, la dystrophie cornéenne endothéliale de Fuchs, la dystrophie cornéenne polymorphe postérieure, le syndrome endothélial iridocornéen, la dystrophie endothéliale cornéenne héréditaire congénitale, l'endothéliite à cytomégalovirus, l'endothéliite herpétique, le syndrome d'exfoliation, le rejet de greffe endothéliale cornéenne, l'uvéite kératite, la kératite interstitielle, l'endothéliite cornéenne, la perte de cellules endothéliales cornéennes après greffe de cornée, la lésion cornéenne après chirurgie intraoculaire, la lésion cornéenne induite par une attaque glaucomateuse, la lésion cornéenne causée par l'utilisation à long terme de lentilles de contact, le traumatisme cornéen, le traumatisme cornéen intrapartum, la kératopathie bulleuse, la kératite ponctuée superficielle, l'érosion cornéenne, l'ulcère cornéen, la sécheresse oculaire, la kératoconjonctivite sèche ou l'ulcère marginal cornéen.

9. Composition à utiliser selon la revendication 5, dans laquelle la maladie cornéenne est la dystrophie cornéenne endothéliale de Fuchs, la kératopathie bulleuse, l'érosion cornéenne, la sécheresse oculaire ou le traumatisme cornéen.

10. Composition à utiliser selon l'une quelconque des revendications 1 à 9, dans laquelle la composition est un collyre.

11. Composition à utiliser selon l'une quelconque des revendications 1 à 9, dans laquelle la composition est une solution d'irrigation oculaire ou une composition à ajouter à une solution d'irrigation oculaire.

12. Composition pharmaceutique à utiliser pour le traitement ou la prévention d'une maladie cornéenne, comprenant un composé de formule (I) : 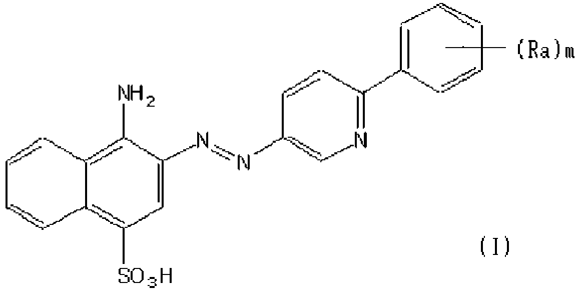 dans laquelle
Ra est choisi dans le groupe consistant en un halo, un hydroxy, un alkyle, un alkyle substitué par un halo, un aryle, un aryle substitué par un halo ou un alkyle, un alcoxy, un alcoxy substitué par un hydroxy ou un carboxy, un aryloxy, un aryloxy substitué par un halo ou un alkyle, CHO, C(O)-alkyle, C(O)-aryle, C(O)-alkyl-carboxyle, ester de C(O)-alkylène-carboxy, et cyano, et
m est un entier choisi de 0 à 4,
ou un ester, oxyde, sel ou solvate pharmaceutiquement acceptable de celui-ci.

13. Composition à utiliser pour une irrigation oculaire, comprenant un composé de formule (I) : 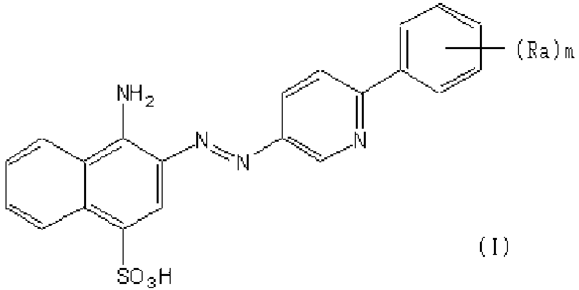 dans laquelle
Ra est choisi dans le groupe consistant en un halo, un hydroxy, un alkyle, un alkyle substitué par un halo, un aryle, un aryle substitué par un halo ou un alkyle, un alcoxy, un alcoxy substitué par un hydroxy ou un carboxy, un aryloxy, un aryloxy substitué par un halo ou un alkyle, CHO, C(O)-alkyle, C(O)-aryle, C(O)-alkyl-carboxyle, ester de C(O)-alkylène-carboxy, et cyano, et
m est un entier choisi de 0 à 4,
ou un ester, oxyde, sel ou solvate pharmaceutiquement acceptable de celui-ci.

14. Utilisation d'une composition pour une conservation d'un greffon cornéen, la composition comprenant un composé de formule (I) : 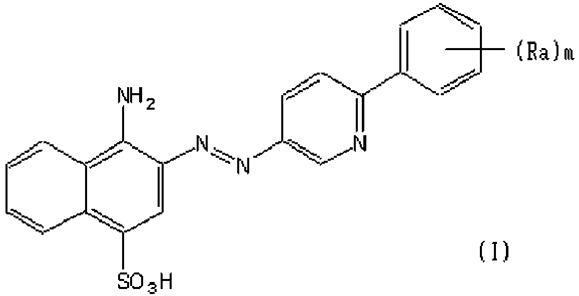 dans laquelle
Ra est choisi dans le groupe consistant en un halo, un hydroxy, un alkyle, un alkyle substitué par un halo, un aryle, un aryle substitué par un halo ou un alkyle, un alcoxy, un alcoxy substitué par un hydroxy ou un carboxy, un aryloxy, un aryloxy substitué par un halo ou un alkyle, CHO, C(O)-alkyle, C(O)-aryle, C(O)-alkyl-carboxyle, ester de C(O)-alkylène-carboxy, et cyano, et
m est un entier choisi de 0 à 4,
ou un ester, oxyde, sel ou solvate pharmaceutiquement acceptable de celui-ci.

15. Procédé de conservation ou de protection d'un greffon cornéen, comprenant une conservation d'un greffon cornéen dans une solution de conservation cornéenne comprenant un composé de formule (I) : 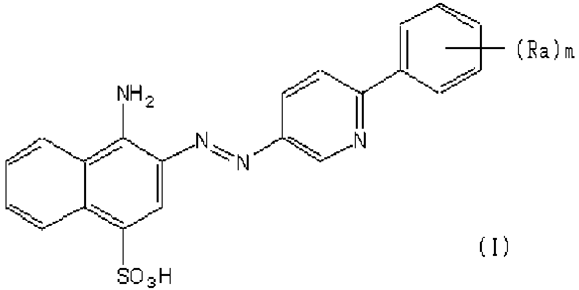 dans laquelle
Ra est choisi dans le groupe consistant en un halo, un hydroxy, un alkyle, un alkyle substitué par un halo, un aryle, un aryle substitué par un halo ou un alkyle, un alcoxy, un alcoxy substitué par un hydroxy ou un carboxy, un aryloxy, un aryloxy substitué par un halo ou un alkyle, CHO, C(O)-alkyle, C(O)-aryle, C(O)-alkyl-carboxyle, ester de C(O)-alkylène-carboxy, et cyano, et
m est un entier choisi de 0 à 4,
ou un ester, oxyde, sel ou solvate pharmaceutiquement acceptable de celui-ci.
